# EUROPEAN PATENT SPECIFICATION

(11) **EP 2 831 291 B1**
(45) Date of publication and mention of the grant of the patent: **24.04.2019**
(21) Application number: 13769600.1
(22) Date of filing: 28.03.2013
(51) Int. Cl.: C12N 5/077, C14C 3/00, C14B 1/30, C14B 1/56, C14B 7/00, B32B 9/02

(54) **ENGINEERED LEATHER AND METHODS OF MANUFACTURE THEREOF**
MANIPULIERTES LEDER UND VERFAHREN ZUR HERSTELLUNG DAVON
CUIR DE SYNTHÈSE ET PROCÉDÉS DE FABRICATION DE CELUI-CI

(30) Priority: 28.03.2012 US 201261616888 P
(43) Date of publication of application: 04.02.2015
(73) Proprietor: Modern Meadow, Inc., Brooklyn, NY 11220 (US)
(72) Inventor: FORGACS, Gabor, Columbia, MO 65211-3460 (US); MARGA, Francoise, Columbia, MO 65211-3460 (US); JAKAB, Karoly, Columbia, MO 65211-3460 (US)
(74) Representative: J A Kemp
(86) International application number: PCT/US2013/034486
(87) International publication number: WO 2013/149083

(56) References cited:
- EP-A1- 1 589 098
- WO-A1-2009/070720
- WO-A1-2011/051983
- JP-A- H0 617 378
- US-A1- 2003 190 438
- US-A1- 2009 069 893
- US-A1- 2012 023 777
- JAKAB K ET AL: "Tissue engineering by self-assembly and bio-printing of living cells", BIOFABRICATION, vol. 2, no. 2, 022001, 2 June 2010 (2010-06-02), pages 1-15, XP055148027, DOI: 10.1088/1758-5082/2/2/022001

## Description

### BACKGROUND

Leather is used in a vast variety of applications, including furniture upholstery, clothing, shoes, luggage, handbag and accessories, and automotive applications. Currently, skins of animals are used as raw materials for natural leather. However, skins from livestock pose environmental concerns because raising livestock requires enormous amounts of feed, pasteurland, water, and fossil fuel. Livestock also produces significant pollution for the air and waterways.

In addition, use of animal skins to produce leather is objectionable to socially conscious individuals. The global leather industry slaughters more than a billion animals per year. Most of the leather comes from countries with no animal welfare laws or have laws that go largely or completely unenforced. Leather produced without killing animals would have tremendous fashion novelty and appeal.

Although synthetic leather was developed to address some of these concerns, it lacks the quality, durability, and prestige of natural leather. Thus far, scientifically sound and industrially feasible processes have not been developed to produce natural leather. Accordingly, there is a need for a solution to demands for alternative to leather produced from live animals.

Natural leather is typically a durable and flexible material created by the tanning of animal rawhide and skin, often cattle hide. Tanning is generally understood to be the process of treating the skins of animals to produce leather. Tanning may be performed in any number of well-understood ways, including vegetable tanning (e.g., using tannin), chrome tanning (chromium salts including chromium sulfate), aldehyde tanning (using glutaraldehyde or oxazolidine compounds), syntans (synthetic tannins, using aromatic polymers), and the like.

Natural leather is typically prepared in three main parts: preparatory stages, tanning, and crusting. Surface coating may also be included. The preparatory stages prepare the hide/skin for tanning, and unwanted raw skin components are removed. The preparatory stages may include: preservation, soaking (rehydrating), liming, de-hairing, fleshing (removing subcutaneous material), splitting, re-liming, deliming (to remove de-hairing and liming chemicals), bating (protein proteolysis), degreasing, frizzing, bleaching, pickling (changing pH), de-pickling, etc.

Tanning is performed to convert proteins in the hide/skin into a stable material that will not putrefy, while allowing the material to remain flexible. Chromium is the most commonly used tanning material. The pH of the skin/hide may be adjusted (e.g., lowered, e.g. to pH 2.8-3.2) to enhance the tanning; following tanning the pH may be raised ("basification" to a slightly higher level, e.g., pH 3.8-4.2).

Crusting refers to the post-tanning treatment that may include coloring (dying), thinning, drying or hydrating, and the like. Examples of crusting techniques include: wetting (rehydrating), sammying (drying), splitting (into thinner layers), shaving, neutralization (adjusting pH to more neutral level), retanning, dyeing, fatliquoring, filling, stuffing, stripping, whitening, fixation of unbound chemicals, setting, conditioning, softening, buffing, etc.

In practice, the process of converting animal skin into leather may include sequential steps such as: unhairing/dehairing, liming, deliming and bateing, pickling, tanning, neutralizing/Dyeing and Fat liquoring, drying and finishing. The dehairing process may chemically remove the hair (e.g., using an alkali solution), while the liming step (e.g., using an alkali and sulfide solution) may further complete the hair removal process and swell ("open up") the collagen. During tanning, the skin structure may be stabilized in the "open" form by replacing some of the collagen with complex ions of chromium. Depending on the compounds used, the colour and texture of the leather may change. Tanned leather may be much more flexible than an untreated hide, and also more durable.

Skin, or animal hide, is formed primarily of collagen, a fibrous protein. Collagen is a generic term for a family of at least 28 distinct collagen types; animal skin is typically type 1 collagen (so the term collagen is typically assumed to be type 1 collagen), although other types of collagen may be used in forming leather. Collagens are characterized by a repeating triplet of amino acids, -(Gly-X-Y)*ₙ*-, so that approximately one-third of the amino acid residues are in collagen are glycine. X is often proline and Y is often hydroxyproline. Thus, the structure of collagen may consist of twined triple units of peptide chains of differing lengths. Different animals may produce different amino acid compositions of the collagen, which may result in different properties (and differences in the resulting leather). Collagen fiber monomers may be produced from alpha-chains of about 1050 amino acids long, so that the triple helix takes the form of a rod of about 300 nm long, with a diameter of 1.5 nm. In the production of extracellular matrix by fibroblast skin cells, triple helix monomers may be synthesized and the monomers may self-assemble into a fibrous form. These triple helices may be held together by salt links, hydrogen bonding, hydrophobic bonding, and covalent bonding. Triple helices can be bound together in bundles called fibrils, and fibril bundles come together to create fibers. Fibers typically divide and join with each other throughout a layer of skin. Variations of the crosslinking or linking may provide strength to the material. Fibers may have a range of diameters. In addition to type I collagen, skin (hides) may include other types of collagen as well, including type III collagen (reticulin), type IV collagen, and type VII collagen.

Described herein are artificial leathers that replicate much of the structures and properties of natural leathers, but may be processed in a much simpler manner, and may address many of the problems of natural and previously-described artificial leathers identified above.

### SUMMARY OF THE DISCLOSURE

According to the present invention there is provided the method of claim 1. Additional aspects of the invention are set out in the dependent claims.

Disclosed herein are engineered animal skin, hide, and leather, and methods of producing the same. In certain embodiments, disclosed herein is an engineered animal skin, hide, or leather comprising a plurality of layers of extracellular matrix, ECM, (e.g., collagen) formed from cultured cells. For example, the cultured cells (with and without ECM) may be within multicellular bodies comprising one or more types of skin cells, wherein said animal cells are cultured *in vitro.* In certain embodiments, disclosed herein is an engineered animal skin, hide, or leather comprising a plurality of layers of animal cells comprising one or more types of skin cells, wherein said animal cells are cultured *in vitro.* In certain embodiments, the animal cells provided herein are non-human cells. It should be understood that although skin cells are described and illustrated herein, any collagen-producing cell (e.g., cell that can produce or be induced to produce collagen ECM) may be used with any of the methods described herein to produce the engineered leather described. Collagen ECM producing cells may include muscle cells (including smooth muscle cells) and the like.

The engineered leather described herein, e.g., using a process such as those descried herein, may be grossly similar (if not identical) to natural leathers. However, these engineered leathers may include numerous differences rising from the method of formation, using cultured cells. For example, the sheets of extracellular matrix formed and stacked (and completely or partially fused) as described herein may be formed of the cultured skin cells so that each layer is substantially homogenous within the layer. Unlike natural leathers, the engineered leathers described herein may be completely free of muscle (e.g., papillary muscle), hair and hair follicles, blood vessels, and the like, as the material forming the leather is grown from cultured cells. During the formation process, the engineered leather may be formed to precise dimensions, including thickness, and without the need to prepare the material as is necessary with natural hides, including liming, de-hairing, splitting, fleshing, etc.

In some variations, the engineered leather is formed from layers themselves formed by biofabrication. For example, in certain embodiments, each layer of multicellular bodies provided herein is deposited. In some embodiments, the deposition of each layer of multicellular bodies is automated. In some embodiments, each layer of multicellular bodies provided herein is deposited without a structural scaffold.

In some embodiments the biofabrication methods use multicellular bodies. For example, a plurality of layers may be formed of multicellular bodies provided that comprise an animal epidermis, basement membrane, dermis, hypodermis, scale, scute, osteoderm, or a combination thereof. In some embodiments, animal epidermis provided herein comprises stratum corneum, stratum lucidum, stratum granulosum, stratum spinosum, stratum germinativum, stratum basale, or a combination thereof.

For example, described herein are engineered leather comprising: a body having a volume; wherein the body comprises a plurality of layers, wherein each layer comprises collagen released by cultured cells; wherein the body is completely devoid of hair, hair follicles, and blood vessels.

The layers may be at least partially fused. In some variations, each layer comprises a homogenous distribution of collagen within the layer, which is a result of the method of fabrication by forming sheets and layering them atop each other to produce the body that is tanned to form the engineered leather.

The collagen-producing cells may comprise epithelial cells, fibroblasts, keratinocytes, corneocytes, melanocytes, Langerhans cells, basal cells, or a combination thereof. The epithelial cells may comprise squamous cells, cuboidal cells, columnar cells, basal cells, or a combination thereof. The fibroblasts may be dermal fibroblasts. The keratinocytes may be epithelial keratinocytes, basal keratinocytes, proliferating basal keratinocytes, differentiated suprabasal keratinocytes, or a combination thereof. The present invention also includes the engineered leather of claim 1, further comprising an extra-cellular matrix or connective tissue.

In some variations, the engineered leather further comprises one or more components selected from the group consisting of keratin, elastin, gelatin, proteoglycan, dermatan sulfate proteoglycan, glycosoaminoglycan, fibronectin, laminin, dermatopontin, lipid, fatty acid, carbohydrate, and a combination thereof.

The animal cells may be derived from mammals selected from the group consisting of antelope, bear, beaver, bison, boar, camel, caribou, cat, cattle, deer, dog, elephant, elk, fox, giraffe, goat, hare, horse, ibex, kangaroo, lion, llama, lynx, mink, moose, oxen, peccary, pig, rabbit, seal, sheep, squirrel, tiger, whale, wolf, yak, and zebra, and a combination thereof. The animal cells may be derived from reptiles selected from the group consisting of turtle, snake, crocodile, and alligator, or combinations thereof. The animal cells may be derived from birds selected from the group consisting of chicken, duck, emu, goose, grouse, ostrich, pheasant, pigeon, quail, and turkey, or combinations thereof. The animal cells may be derived from fish selected from the group consisting of anchovy, bass, catfish, carp, cod, eel, flounder, fugu, grouper, haddock, halibut, herring, mackerel, mahi mahi, manta ray, marlin, orange roughy, perch, pike, pollock, salmon, sardine, shark, snapper, sole, stingray, swordfish, tilapia, trout, tuna, and walleye, or combinations thereof.

In general, the engineered leather may be formed without the need for a structural scaffold.

At least one of the layers of the engineered leather may comprise a ratio of animal fibroblasts to animal keratinocytes between about 20:1 to about 3:1. The engineered leather layers may be substantially free of non-differentiated keratinocytes, fibroblasts, or epithelial cells.

In general, the engineered leather may be patterned. For example, the engineered leather may be patterned after a skin pattern of an animal selected from antelope, bear, beaver, bison, boar, camel, caribou, cat, cattle, deer, dog, elephant, elk, fox, giraffe, goat, hare, horse, ibex, kangaroo, lion, llama, lynx, mink, moose, oxen, peccary, pig, rabbit, seal, sheep, squirrel, tiger, whale, wolf, yak, zebra, turtle, snake, crocodile, alligator, dinosaur, frog, toad, salamander, newt, chicken, duck, emu, goose, grouse, ostrich, pheasant, pigeon, quail, turkey, anchovy, bass, catfish, carp, cod, eel, flounder, fugu, grouper, haddock, halibut, herring, mackerel, mahi mahi, manta ray, marlin, orange roughy, perch, pike, pollock, salmon, sardine, shark, snapper, sole, stingray, swordfish, tilapia, trout, tuna, walleye, and a combination thereof.

In general, the engineered leather may include layers having a thickness that is characterized as adapted to allow diffusion to sufficiently support the maintenance and growth of said animal cells in culture. The thickness of each said sheet may be about 50 µm to about 200 µm. For example, the thickness of each said layer may be about 50 µm to about 150 µm.

Any appropriate number of layers may be used, and may be selected based on the desired thickness. As the sheets are formed and layered atop one another, in some variations, the cells in one of the layers may be killed or allowed to die. For example, cells that are in a sheet that is already layered in the body may be allowed to die (e.g., for lack of nutrients) while cells in the top or outer layer(s) live and may help adhere (e.g., by release/remodeling of ECM) to the adjacent layers. For example, an engineered leather may include a plurality of layers, e.g., comprising 2 to about 50 layers, 2 to about 40 layers, 2 to about 30 layers, etc.

Any of the engineered leather described herein may be colored, e.g., comprising one or more colorants or pigments, or patterned.

Also described herein are methods of producing engineered leather. In general, these methods allow the production of engineered leather to any desired thickness from cultured collagen-producing (e.g., skin) cells, eliminating the need for some of the more resource-intensive and polluting steps associated with traditional leather making, including, e.g., de-hairing, soaking, fleshing, liming/deliming, splitting, and bleaching.

For example in some variations the method includes: culturing one or more types of skin cells in vitro; forming a plurality of sheets of the one or more types of skin cells and extracellular matrix material including collagen; layering the plurality of sheets to form a body having a volume; and processing the body by tanning.

In some variations the methods include forming the sheets or layers by biofabrication. For example, the method may also include preparing a plurality of elongate or spherical multicellular bodies comprising said one or more types of skin cells, wherein the cells are cohered to one another. These multicellular bodies may be used in a biofabrication technique of positioning these multicellular bodies in a layer and allowing them to fuse and form extracellular matrix, and particularly collagen. For example, forming a plurality of sheets may comprise forming a plurality of planar layers by adjacently arranging a plurality of elongate multicellular bodies, wherein said elongate multicellular bodies are fused to form a planar layer. The step of arranging may comprise placing multicellular bodies on a support substrate that allows the multicellular bodies to fuse to form a substantially planar layer.

The structure supporting the sheet as it is formed (e.g., a support substrate, such as a culture dish) may be permeable to fluids and nutrients to allow cell culture media to contact all surfaces of said layer.

Thus, in some variations, the forming of the sheets comprises automated deposition of multicellular bodies into said layers without a structural scaffold. Multicellular bodies may be arranged horizontally and/or vertically adjacent to one another. Fusing of multicellular bodies to form the sheet may take place over about 2 hours to about 24 hours. Extracellular matrix (e.g., collagen) may be laid down over the same time period, or over longer time (e.g., one day to 3 days, one day to four days, one day to 5 days, one day to six days, one day to seven days, etc.). When forming sheets by biofabrication, elongate multicellular bodies of skin cells may be formed having the same lengths. For example, the elongate multicellular bodies may have a length ranging from about 1 mm to about 10 m, about 1 cm to about 1 m, about 1 cm to about 50 cm, etc.

In any of the methods of forming the engineered leather described herein the method may include one or more processing steps in addition to the tanning step. The tanning step may be performed in any appropriate manner, such as chrome tanning (using chromium salt). The method may further include processing the layered body using one or more additional processing steps. Additional processing steps may include: preserving, soaking, bating, pickling, depickling, thinning, retanning, lubricating, crusting, wetting, sammying, shaving, rechroming, neutralizing, dyeing, fatliquoring, filling, stripping, stuffing, whitening, fixating, setting, drying, conditioning, milling, staking, buffing, finishing, oiling, brushing, padding, impregnating, spraying, roller coating, curtain coating, polishing, plating, embossing, ironing, glazing, and tumbling.

The method of forming engineered leather may use any appropriate skin cell(s). For example, the skin cells may comprise epithelial cells, fibroblasts, keratinocytes, corneocytes, melanocytes, Langerhans cells, basal cells, or a combination thereof. The epithelial cells may comprise squamous cells, cuboidal cells, columnar cells, basal cells, or a combination thereof. The fibroblasts may be dermal fibroblasts. The keratinocytes may be epithelial keratinocytes, basal keratinocytes, proliferating basal keratinocytes, differentiated suprabasal keratinocytes, or a combination thereof.

In some variations, the step of forming the plurality of sheets comprises forming a plurality of sheets of the one or more types of skin cells and extracellular matrix material including collagen and one or more components selected from the group consisting of: keratin, elastin, gelatin, proteoglycan, dermatan sulfate proteoglycan, glycosoaminoglycan, fibronectin, laminin, dermatopontin, lipid, fatty acid, carbohydrate, and a combination thereof.

Forming the plurality of sheets may comprise forming a plurality of sheets of the one or more types of skin cells in a ratio of animal fibroblasts to animal keratinocytes of about 20:1 to about 2:1. In general, the skin cells used may be substantially free of non-differentiated keratinocytes, fibroblasts, or epithelial cells.

The collagen-producing (e.g., skin) cells may be derived from mammals selected from the group consisting of antelope, bear, beaver, bison, boar, camel, caribou, cat, cattle, deer, dog, elephant, elk, fox, giraffe, goat, hare, horse, ibex, kangaroo, lion, llama, lynx, mink, moose, oxen, peccary, pig, rabbit, seal, sheep, squirrel, tiger, whale, wolf, yak, and zebra, and a combination thereof. The animal skin cells may be derived from reptiles selected from the group consisting of turtle, snake, crocodile, and alligator, or combinations thereof. The animal skin cells are derived from birds selected from the group consisting of chicken, duck, emu, goose, grouse, ostrich, pheasant, pigeon, quail, and turkey, or combinations thereof. The animal skin cells are derived from fish selected from the group consisting of anchovy, bass, catfish, carp, cod, eel, flounder, fugu, grouper, haddock, halibut, herring, mackerel, mahi mahi, manta ray, marlin, orange roughy, perch, pike, pollock, salmon, sardine, shark, snapper, sole, stingray, swordfish, tilapia, trout, tuna, and walleye, or combinations thereof.

As mentioned, the engineered leather described herein may be patterned. For example, the method may include aligning the skin cells to form a pattern.

In general, the step of forming the layers may comprise automated deposition of multicellular bodies to form the sheets.

The sheets or layers formed may be of any appropriate thinness/thickness. In some variations the thickness of each layer of the plurality of sheets is characterized by a thickness adapted to allow diffusion to sufficiently support the maintenance and growth of said animal cells in culture. For example, the thickness of each said sheet may be about 50 µm to about 200 um, about 50 µm to about 150 um, about 50 µm to about 100 um, etc.

Similarly, any appropriate number of layers may be selected, which may determine the thickness of the engineered lather. For example, the engineered leather may comprise 2 to about 50 layers, 2 to about 40 layers, 2 to about 30 layers, etc.

Any of the methods described herein may also include coloring or dying the engineered leather. For example, the method may include dying the layered body using one or more colorants or pigments.

### BRIEF DESCRIPTION OF THE DRAWINGS

Fig. 1 depicts a non-limiting example of an elongate multicellular body; in this case, an elongate multicellular body 1 with width Wl that is approximately equal to height HI and length LI that is substantially greater than width Wl or height HI.
Fig. 2 depicts a non-limiting example of a substantially spherical multicellular body; in this case, a substantially spherical multicellular body 2 with width Wl that is approximately equal to height HI.
Fig. 3 depicts a non-limiting example of an elongate multicellular body; in this case, an elongate multicellular body 1 on a support substrate 3.
Fig. 4 depicts a non-limiting example of a substantially spherical multicellular body; in this case, a substantially spherical multicellular body 2 on a support substrate 3.
Fig. 5 depicts a non-limiting example of one method of making the multicellular bodies illustrated in Figs. 1-4; in this case, a method involving transferring a mixed cell pellet 4 into a capillary tube 5.
Fig. 6 depicts a non-limiting example of a plurality of elongate multicellular bodies; in this case, a plurality of elongate multicellular bodies 1 are laid adjacently onto a support substrate 3 such that they are allowed to fuse.
Fig. 7 depicts a non-limiting example of a plurality of substantially spherical multicellular bodies; in this case, a plurality of substantially spherical multicellular bodies 2 lay adjacently onto a support substrate 3 such that they are allowed to fuse.
Fig. 8 depicts a non-limiting example of one method of making a layer comprising a plurality of elongate multicellular bodies; in this case, a method involving extruding multicellular bodies 6 from a pressure-operated mechanical extruder comprising a capillary tube 5 onto a support substrate 3.
Fig. 9 depicts a non-limiting example of one method of making engineered animal skin, hide, or leather; in this case, a method involving laying more than one layer, comprising a plurality of elongate multicellular bodies 7, 8, adjacently onto a support substrate 3.
Fig. 10 depicts a non-limiting example of one method of making engineered animal skin, hide, or leather; in this case, a method involving laying more than one layer, comprising a plurality of elongate multicellular bodies 9 and a plurality of substantially spherical multicellular bodies 10, adjacently onto a support substrate 3.
Fig. 11 depicts a non-limiting example of one method of making engineered animal skin, hide, or leather; in this case, a method involving stacking more than one layer, wherein layers subsequent to the first are rotated 90 degrees with respect to the layer below.
FIG. 12 illustrates a schematic overview of a method of forming an engineered leather as described herein.

### DETAILED DESCRIPTION

Tissue engineering technology offers new opportunities to produce animal skin, hide, or leather that are not associated with the environmental degradation of raising livestock. Tissue engineering has been defined as an interdisciplinary field that applies the principles of engineering and life sciences toward the development of biological substitutes that restore, maintain, or improve tissue function or a whole organ. Langer R, Vacanti JP, Tissue Engineering, Science 260(5110):920-926 (May 1993).

Tissue engineered products made using traditional materials and methods are limited in size due to the short distances gases and nutrients can diffuse to nourish interior cells. Also, existing techniques fail to provide adequate speed and throughput for mass production of engineered products. As a result, existing tissue engineering methods result in unappealing thin sheets and pastes on a commercially infeasible scale.

Thus, an objective of the animal skin, hide, or leather, and methods of making the same disclosed herein is to provide commercially viable and appealing animal skin, hide, or leather. Another objective is to provide high-throughput methods that reliably, accurately, and reproducibly scale up to commercial levels. Advantages of the animal skin, hide, or leather, and methods of making the same disclosed herein include, but are not limited to, production of customized tissues in a reproducible, high throughput and easily scalable fashion while keeping precise control of pattern formation, particularly in cases of multiple cell types, which may result in engineered animal skin, hide, or leather with appealing appearance, texture, thickness, and durability.

Disclosed herein are engineered animal hide and leather, and methods of producing the same. In certain embodiments, disclosed herein is engineered animal skin, hide, or leather comprising a plurality of layers of animal cells comprising one or more types of skin cells, wherein said animal cells are cultured *in vitro.* In certain embodiments, each layer of animal cells provided herein is biofabricated. In some embodiments, each layer of animal cells provided herein is biofabricated without a structural scaffold.

In certain embodiments, provided herein is a plurality of multicellular bodies comprising an elongated or spherical shape, and one or more types of animal skin cells, wherein the cells are cultured *in vitro* and are cohered to one another within the multicellular body, wherein said multicellular bodies are arranged in one or more substantially planar layers that form engineered animal skin, hide, or leather. In certain embodiments, each layer of animal cells provided herein is biofabricated. In some embodiments, each layer of animal cells provided herein is biofabricated without a structural scaffold.

In certain embodiments, provided herein are methods of producing an engineered animal skin, hide, or leather, comprising culturing *in vitro* animal cells comprising one or more types of skin cells, preparing a plurality of elongate or spherical multicellular bodies comprising said animal cells, wherein the cells are cohered to one another, and forming a plurality of planar layers comprising adjacently arranging a plurality of elongate multicellular bodies, wherein said elongate multicellular bodies are fused to form a planar layer. In certain embodiments, said preparing step comprises biofabrication to position multicellular bodies or said layers. In some embodiments, said preparing step comprises biofabrication to position multicellular bodies or said layers without a structural scaffold.

The term "adjacent," as used herein when referring to arrangement of multicellular bodies, means in contact and on top of, under, or next to, either horizontally or vertically relative to the support substrate.

### B iofabrication

A basic idea underlying classical tissue engineering is to seed living cells into biocompatible and eventually biodegradable scaffold, and then culture the system in a bioreactor so that the initial cell population can expand into a tissue. Classical tissue engineering harbors several shortcomings, especially when applied to the production of animal skin, hide, or leather products. First, the process of seeding cells generally involves contacting a solution of cells with a scaffold such that the cells are trapped within pores, fibers, or other micro structure of the scaffold. This process is substantially random with regard to the placement of cells within the scaffold and the placement of cells relative to each other. Therefore, seeded scaffolds are not immediately useful for production of three-dimensional constructs that exhibit planned or pre-determined placement or patterns of cells or cell aggregates. Second, selection of the ideal biomaterial scaffold for a given cell type is problematic and often accomplished by trial and error. Even if the right biomaterial is available, a scaffold can interfere with achieving high cell density. Moreover, scaffold-based tissue engineering does not easily or reliably scale up to industrial levels.

In some embodiments, the engineered animal skin, hide, leather, layers, and multicellular bodies are made with a method that utilizes a rapid prototyping technology based on three-dimensional, automated, computer-aided deposition of multicellular bodies (e.g., cylinders and spheroids) and a biocompatible support structure (e.g., composed of agarose) by a three-dimensional delivery device (e.g., a biofabricator). As used herein, in some embodiments, the term "engineered," when used to refer to the animal skin, hide, and leather, means that multicellular bodies and/or layers of animal cells are positioned to form engineered animal skin, hide, and leather by a computer-aided device (e.g., a biofabricator) according to a computer script. In further embodiments, the computer script is, for example, one or more computer programs, computer applications, or computer modules. In still further embodiments, three-dimensional tissue structures form through the post-positioning fusion of the multicellular bodies similar to self-assembly phenomena in early morphogenesis.

While a number of methods are available to arrange the multicellular bodies on a support substrate to produce a three-dimensional structure including manual placement, positioning by an automated, computer-aided machine such as a fabricator is advantageous. Advantages of delivery of multicellular bodies with this technology include rapid, accurate, and reproducible placement of multicellular bodies to produce constructs exhibiting planned or pre-determined orientations or patterns of multicellular bodies and/or layers of various compositions. Advantages also include assured high cell density, while minimizing cell damage often associated with other solid freeform fabrication-based deposition methods focused on positioning/placing cells in combination with hydrogels.

The speed and scalability of the techniques and methods disclosed herein can be utilized to design, build, and operate industrial and/or commercial facilities for the production of engineered animal skin, hide, and leather. The engineered animal skin, hide, and leather can be produced, packaged, stored, distributed, marketed, advertised, and sold as, for example, furniture upholstery, clothing, shoes, luggage, handbag and accessories, and automotive applications.

In certain embodiments, animal skin, hide, and leather provided herein are patterned. In some embodiments, the pattern provided herein is a skin pattern of an animal selected from antelope, bear, beaver, bison, boar, camel, caribou, cat, cattle, deer, dog, elephant, elk, fox, giraffe, goat, hare, horse, ibex, kangaroo, lion, llama, lynx, mink, moose, oxen, peccary, pig, rabbit, seal, sheep, squirrel, tiger, whale, wolf, yak, zebra, turtle, snake, crocodile, alligator, dinosaur, frog, toad, salamander, newt, chicken, duck, emu, goose, grouse, ostrich, pheasant, pigeon, quail, turkey, anchovy, bass, catfish, carp, cod, eel, flounder, fugu, grouper, haddock, halibut, herring, mackerel, mahi mahi, manta ray, marlin, orange roughy, perch, pike, pollock, salmon, sardine, shark, snapper, sole, stingray, swordfish, tilapia, trout, tuna, walleye, and a combination thereof.

In certain embodiments, the engineered animal skin, hide, or leather provided herein further comprises one or more colorants or pigments.

### Cells

Many self-adhering cell types may be used to form the multicellular bodies, layers, and engineered skin, hide, and leather products described herein. In some embodiments, the engineered animal skin, hide, and leather products are designed to resemble traditional animal skin, hide, and leather products and the cell types are chosen to approximate those found in traditional animal skin, hide, and leather products. In further embodiments, the engineered animal skin, hide, and leather products, layers, and multicellular bodies include animal epidermis, basement membrane, dermis, hypodermis, scale, scute, osteoderm, or a combination thereof. In some embodiments, animal epidermis provided herein comprises stratum corneum, stratum lucidum, stratum granulosum, stratum spinosum, stratum germinativum, stratum basale, or a combination thereof. In some embodiments, animal dermis provided herein comprises stratum papillare, stratum reticulare, or a combination thereof. In some embodiments, animal scale provided herein comprises placoid scale, cosmoid scale, ganoid scale, elasmoid scale, cycloid scale, ctenoid scale, crenate scale, spinoid scale, or a combination thereof.

In certain embodiments, animal cells provided herein comprise epithelial cells, fibroblasts, keratinocytes, corneocytes, melanocytes, Langerhans cells, basal cells, or a combination thereof. In some embodiments, epithelial cells provided herein comprise squamous cells, cuboidal cells, columnar cells, basal cells, or a combination thereof. In some embodiments, fibroblasts provided herein are dermal fibroblasts. In some embodiments, keratinocytes provided herein are epithelial keratinocytes, basal keratinocytes, proliferating basal keratinocytes, differentiated suprabasal keratinocytes, or a combination thereof.

In certain embodiments, the ratio of animal fibroblasts to animal keratinocytes provided herein is between about 20:1 to about 3:1. In some embodiments, the ratio of animal fibroblasts to animal keratinocytes provided herein is between about 20:1 to about 4:1. In some embodiments, the ratio of animal fibroblasts to animal keratinocytes provided herein is between about 20:1 to about 5:1. In some embodiments, the ratio of animal fibroblasts to animal keratinocytes provided herein is between about 20:1 to about 10:1. In some embodiments, the ratio of animal fibroblasts to animal keratinocytes provided herein is between about 20:1 to about 15:1. In some embodiments, the ratio of animal fibroblasts to animal keratinocytes provided herein is about 25:1. In some embodiments, the ratio of animal fibroblasts to animal keratinocytes provided herein is about 24:1. In some embodiments, the ratio of animal fibroblasts to animal keratinocytes provided herein is about 23:1. In some embodiments, the ratio of animal fibroblasts to animal keratinocytes provided herein is about 22:1. In some embodiments, the ratio of animal fibroblasts to animal keratinocytes provided herein is about 21:1. In some embodiments, the ratio of animal fibroblasts to animal keratinocytes provided herein is about 20:1. In some embodiments, the ratio of animal fibroblasts to animal keratinocytes provided herein is about 19:1. In some embodiments, the ratio of animal fibroblasts to animal keratinocytes provided herein is about 18:1. In some embodiments, the ratio of animal fibroblasts to animal keratinocytes provided herein is about 17:1. In some embodiments, the ratio of animal fibroblasts to animal keratinocytes provided herein is about 16:1. In some embodiments, the ratio of animal fibroblasts to animal keratinocytes provided herein is about 15:1. In some embodiments, the ratio of animal fibroblasts to animal keratinocytes provided herein is about 14:1. In some embodiments, the ratio of animal fibroblasts to animal keratinocytes provided herein is about 13:1. In some embodiments, the ratio of animal fibroblasts to animal keratinocytes provided herein is about 12:1. In some embodiments, the ratio of animal fibroblasts to animal keratinocytes provided herein is about 11:1. In some embodiments, the ratio of animal fibroblasts to animal keratinocytes provided herein is about 10:1. In some embodiments, the ratio of animal fibroblasts to animal keratinocytes provided herein is about 9:1. In some embodiments, the ratio of animal fibroblasts to animal keratinocytes provided herein is about 8:1. In some embodiments, the ratio of animal fibroblasts to animal keratinocytes provided herein is about 7:1. In some embodiments, the ratio of animal fibroblasts to animal keratinocytes provided herein is about 6:1. In some embodiments, the ratio of animal fibroblasts to animal keratinocytes provided herein is about 5:1. In some embodiments, the ratio of animal fibroblasts to animal keratinocytes provided herein is about 4:1. In some embodiments, the ratio of animal fibroblasts to animal keratinocytes provided herein is about 3:1. In some embodiments, the ratio of animal fibroblasts to animal keratinocytes provided herein is about 2:1.

In certain embodiments, animal cells provided herein are substantially free of non-differentiated keratinocytes, fibroblasts, or epithelial cells.

In other embodiments, the engineered animal skin, hide, or leather products include neural cells, connective tissue (including bone, cartilage, cells differentiating into bone forming cells and chondrocytes, and lymph tissues), epithelial cells (including endothelial cells that form linings in cavities and vessels or channels, exocrine secretory epithelial cells, epithelial absorptive cells, keratinizing epithelial cells, and extracellular matrix secretion cells), and undifferentiated cells (such as embryonic cells, stem cells, and other precursor cells), among others.

In certain embodiments, engineered animal skin, hide, or leather further comprises an extracellular matrix or connective tissue. In certain embodiments, engineered animal skin, hide, or leather further comprises one or more components selected from the group consisting of collagen, keratin, elastin, gelatin, proteoglycan, dermatan sulfate proteoglycan, glycosoaminoglycan, fibronectin, laminin, dermatopontin, lipid, fatty acid, carbohydrate, and a combination thereof.

In some embodiments, the cells used to form a multicellular body are obtained from a live animal and cultured as a primary cell line. For example, in further embodiments, the cells are obtained by biopsy and cultured *ex vivo.* In other embodiments, the cells are obtained from commercial sources.

In certain embodiments, the multicellular bodies, layers comprising multicellular bodies, and engineered animal skin, hide, or leather products comprise animal cells derived from, by way of non-limiting examples, mammals, birds, reptiles, fish, crustaceans, mollusks, cephalopods, insects, non-arthropod invertebrates, and combinations thereof. In some embodiments, the animal cells are human cells.

In certain embodiments, the animal cells provided herein are non-human cells. In some embodiments, suitable cells are derived from mammals such as antelope, bear, beaver, bison, boar, camel, caribou, cat, cattle, deer, dog, elephant, elk, fox, giraffe, goat, hare, horse, ibex, kangaroo, lion, llama, lynx, mink, moose, oxen, peccary, pig, rabbit, seal, sheep, squirrel, tiger, whale, wolf, yak, and zebra, or combinations thereof. In some embodiments, suitable cells are derived from birds such as chicken, duck, emu, goose, grouse, ostrich, pheasant, pigeon, quail, and turkey, or combinations thereof.

In some embodiments, suitable cells are derived from reptiles such as turtle, snake, crocodile, and alligator, or combinations thereof.

In some embodiments, suitable cells are derived from fish such as anchovy, bass, catfish, carp, cod, eel, flounder, fugu, grouper, haddock, halibut, herring, mackerel, mahi mahi, manta ray, marlin, orange roughy, perch, pike, pollock, salmon, sardine, shark, snapper, sole, stingray, swordfish, tilapia, trout, tuna, and walleye, or combinations thereof.

In some embodiments, suitable cells are derived from amphibians such as frog, toad, salamander, newt, or combinations thereof.

In some embodiments, suitable cells are derived from crustaceans such as crab, crayfish, lobster, prawn, and shrimp, or combinations thereof.

In some embodiments, suitable cells are derived from mollusks such as abalone, clam, conch, mussel, oyster, scallop, and snail, or combinations thereof.

In some embodiments, suitable cells are derived from cephalopods such as cuttlefish, octopus, and squid, or combinations thereof.

In some embodiments, suitable cells are derived from insects such as ants, bees, beetles, butterflies, cockroaches, crickets, damselflies, dragonflies, earwigs, fleas, flies, grasshoppers, mantids, mayflies, moths, silverfish, termites, wasps, or combinations thereof.

In some embodiments, suitable cells are derived from non-arthropod invertebrates (e.g., worms) such as flatworms, tapeworms, flukes, threadworms, roundworms, hookworms, segmented worms (e.g., earthworms, bristle worms, etc.), or combinations thereof.

### Multicellular bodies

Disclosed herein are multicellular bodies including a plurality of living animal cells wherein the cells are adhered and/or cohered to one another. In some embodiments, a multicellular body comprises a plurality of cells adhered and/or cohered together in a desired three-dimensional shape with viscoelastic consistency and sufficient integrity for easy manipulation and handling during a bioengineering process, such as tissue engineering. In some embodiments, sufficient integrity means that the multicellular body, during the subsequent handling, is capable of retaining its physical shape, which is not rigid, but has a viscoelastic consistency, and maintaining the vitality of the cells.

In some embodiments, a multicellular body is homocellular. In other embodiments, a multicellular body is heterocellular. In homocellular multicellular bodies, the plurality of living cells includes a plurality of living cells of a single cell type. Substantially all of the living cells in a homocellular multicellular body are substantially cells of the single cell type. In contrast, a hetero cellular multicellular body includes significant numbers of cells of more than one cell type. The living cells in a heterocellular body may remain unsorted or can "sort out" (e.g., self-assemble) during the fusion process to form a particular internal structure or pattern for the engineered tissue. The sorting of cells is consistent with the predictions of the Differential Adhesion Hypothesis (DAH). The DAH explains the liquid-like behavior of cell populations in terms of tissue surface and interfacial tensions generated by adhesive and cohesive interactions between the component cells. In general, cells can sort based on differences in the adhesive strengths of the cells. For example, cell types that sort to the interior of a heterocellular multicellular body generally have a stronger adhesion strength (and thus higher surface tension) than cells that sort to the outside of the multicellular body.

In some embodiments, the multicellular bodies of the present invention also include one or more extracellular matrix (ECM) components or one or more derivatives of one or more ECM components in addition to the plurality of cells. For example, the multicellular bodies may contain various ECM proteins including, by way of non-limiting examples, gelatin, fibrinogen, fibrin, collagen, fibronectin, laminin, elastin, and proteoglycans. The ECM components or derivatives of ECM components can be added to a cell paste used to form a multicellular body. The ECM components or derivatives of ECM components added to a cell paste can be purified from an animal source, or produced by recombinant methods known in the art. Alternatively, the ECM components or derivatives of ECM components can be naturally secreted by the cells in the multicellular body.

In some embodiments, a multicellular body includes tissue culture medium. In further embodiments, the tissue culture medium can be any physiologically compatible medium and will typically be chosen according to the cell type(s) involved as is known in the art. In some cases, suitable tissue culture medium comprises, for example, basic nutrients such as sugars and amino acids, growth factors, antibiotics (to minimize contamination), etc.

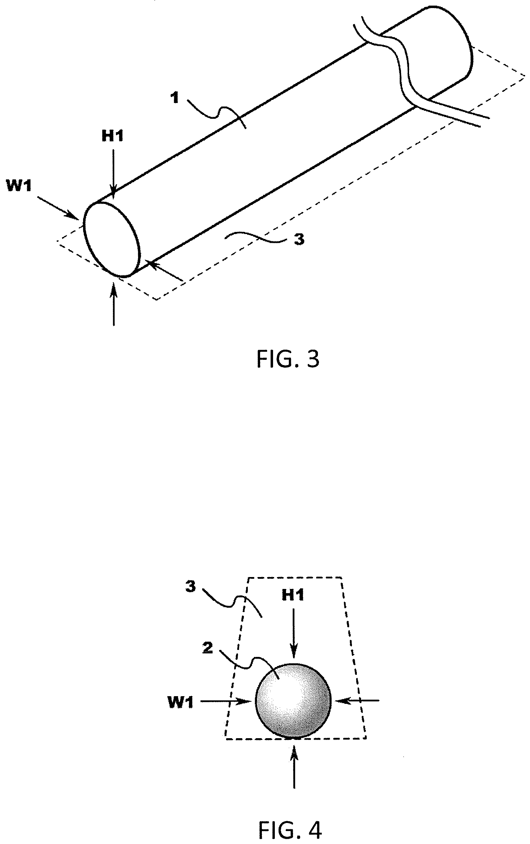

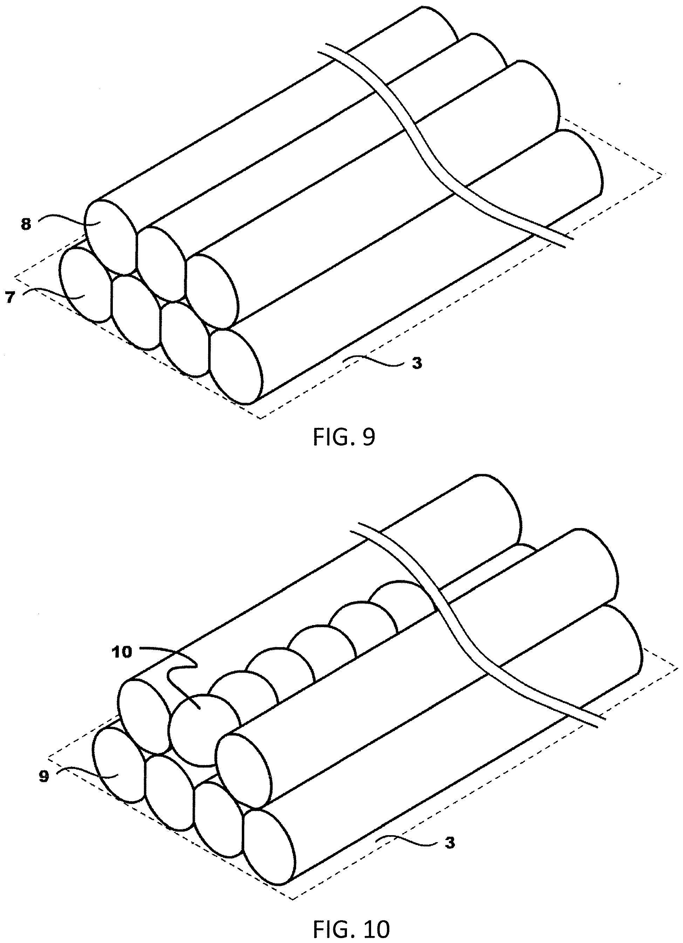

The adhesion and/or cohesion of the cells in a multicellular body is suitably sufficiently strong to allow the multicellular body to retain a three-dimensional shape while supporting itself on a flat surface. For instance, in some cases, a multicellular body supporting itself on a flat substrate may exhibit some minor deformation (e.g., where the multicellular body contacts the surface), however, the multicellular body is sufficiently cohesive to retain a height that is at least one half its width, and in some cases, about equal to the width. In some embodiments, two or more multicellular bodies placed in side-by-side adjoining relation to one another on a flat substrate form a void space under their sides and above the work surface. *See,* e.g., Figs. 3 and 4. In further embodiments, the cohesion of the cells in a multicellular body is sufficiently strong to allow the multicellular body to support the weight of at least one similarly sized and shaped multicellular body when the multicellular body is assembled in a construct in which the multicellular bodies are stacked on top of one another. *See,* e.g., Figs. 9 and 10. In still further embodiments, the adhesion and/or cohesion of the cells in a multicellular body is also suitably sufficiently strong to allow the multicellular body to be picked up by an implement (e.g., a capillary micropipette).

In light of the disclosure provided herein, those of skill in the art will recognize that multicellular bodies having different sizes and shapes are within the scope of the invention. In some embodiments, a multicellular body is substantially cylindrical and has a substantially circular cross section. For example, a multicellular body, in various embodiments, has an elongate shape (e.g., a cylindrical shape) with a square, rectangular, triangular, or other non-circular cross-sectional shape. Likewise, in various embodiments, a multicellular body has a generally spherical shape, a non-elongate cylindrical shape, or a cuboidal shape.

In various embodiments, the diameter of a multicellular body is about 50, 100, 150, 200, 250, 300, 350, 400, 450, 500, 550, 600, 650, 700, 750, 800, 850, 900, 950, 1000 um, or quantifiable increments therein. In some embodiments, a multicellular body is configured to limit cell necrosis caused by inability of oxygen and/or nutrients to diffuse into central portions of the multicellular body. For example, a multicellular body is suitably configured such that none of the living cells therein is more than about 250 µm from an exterior surface of the multicellular body, and more suitably so none of the living cells therein is more than about 200 µm from an exterior of the multicellular body.

In some embodiments, the multicellular bodies are elongate and have differing lengths. In other embodiments, elongate multicellular bodies are of substantially similar lengths. In various embodiments, the length of an elongate multicellular body is about 0.5, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, or 10 mm, or quantifiable increments therein. In other various embodiments, the length of an elongate multicellular body is about 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, or 10 cm, or quantifiable increments therein. In some embodiments, the length of elongate multicellular bodies is chosen to result in a shape and/or size of engineered animal skin, hide, or leather product that approximates that of a traditional animal skin, hide, or leather product.

In certain embodiments, each layer provided herein is characterized by a thickness adapted to allow diffusion to sufficiently support the maintenance and growth of said animal cells in culture. In some embodiments, the thickness of each said layer is about 50 µm to about 1000 µm. In some embodiments, the thickness of each said layer is about 100 µm to about 900 µm. In some embodiments, the thickness of each said layer is about 100 µm to about 800 µm. In some embodiments, the thickness of each said layer is about 100 µm to about 700 µm. In some embodiments, the thickness of each said layer is about 100 µm to about 600 µm. In some embodiments, the thickness of each said layer is about 100 µm to about 500 µm. In some embodiments, the thickness of each said layer is about 150 µm to about 900 µm. In some embodiments, the thickness of each said layer is about 150 µm to about 800 µm. In some embodiments, the thickness of each said layer is about 150 µm to about 700 µm. In some embodiments, the thickness of each said layer is about 150 µm to about 600 µm. In some embodiments, the thickness of each said layer is about 150 µm to about 550 µm. In some embodiments, the thickness of each said layer is about 150 µm to about 500 µm. In some embodiments, the thickness of each said layer is about 200 µm to about 800 µm. In some embodiments, the thickness of each said layer is about 200 µm to about 700 µm. In some embodiments, the thickness of each said layer is about 200 µm to about 600 µm. In some embodiments, the thickness of each said layer is about 200 µm to about 500 µm. In some embodiments, the thickness of each said layer is about 200 µm to about 400 µm. In some embodiments, the thickness of each said layer is about 250 µm to about 700 µm. In some embodiments, the thickness of each said layer is about 250 µm to about 600 µm. In some embodiments, the thickness of each said layer is about 250 µm to about 500 µm. In some embodiments, the thickness of each said layer is about 250 µm to about 450 µm. In some embodiments, the thickness of each said layer is about 250 µm to about 400 µm. In some embodiments, the thickness of each said layer is about 300 µm to about 600 µm. In some embodiments, the thickness of each said layer is about 300 µm to about 500 µm. In some embodiments, the thickness of each said layer is about 300 µm to about 400 µm.

In some embodiments, the plurality of layers provided herein comprises 2 to about 100 layers. In some embodiments, the plurality of layers provided herein comprises 2 to about 90 layers. In some embodiments, the plurality of layers provided herein comprises 2 to about 80 layers. In some embodiments, the plurality of layers provided herein comprises 2 to about 70 layers. In some embodiments, the plurality of layers provided herein comprises 2 to about 60 layers. In some embodiments, the plurality of layers provided herein comprises 2 to about 50 layers. In some embodiments, the plurality of layers provided herein comprises about 10 to about 40 layers. In some embodiments, the plurality of layers provided herein comprises about 10 to about 30 layers. In some embodiments, the plurality of layers provided herein comprises about 20 to about 80 layers. In some embodiments, the plurality of layers provided herein comprises about 20 to about 70 layers. In some embodiments, the plurality of layers provided herein comprises about 20 to about 60 layers. In some embodiments, the plurality of layers provided herein comprises about 20 to about 50 layers. In some embodiments, the plurality of layers provided herein comprises about 20 to about 40 layers. In some embodiments, the plurality of layers provided herein comprises about 30 to about 60 layers. In some embodiments, the plurality of layers provided herein comprises about 30 to about 50 layers. In some embodiments, the plurality of layers provided herein comprises about 30 to about 40 layers. In some embodiments, the plurality of layers provided herein comprises about 40 to about 60 layers. In some embodiments, the plurality of layers provided herein comprises about 10, 20, 30, 40, 50, 60, 70, 80, 90, or 100 layers.

In some embodiments, multicellular bodies provided herein are arranged on a support substrate that allows the multicellular bodies to fuse to form a substantially planar layer. In some embodiments, the support substrate is permeable to fluids and nutrients to allow cell culture media to contact all surfaces of said layer.

In certain embodiments, the elongate multicellular bodies of animal skin cells provided herein are of the same or differing lengths. In some embodiments, the elongate multicellular bodies of animal skin cells provided herein are of arbitrary lengths. In some embodiments, the elongate multicellular bodies have a length ranging from about 1 mm to about 10 m. In some embodiments, the elongate multicellular bodies have a length ranging from about 1 cm to about 10 m. In some embodiments, the elongate multicellular bodies have a length ranging from about 1 cm to about 9 m. In some embodiments, the elongate multicellular bodies have a length ranging from about 1 cm to about 8 m. In some embodiments, the elongate multicellular bodies have a length ranging from about 1 cm to about 7 m. In some embodiments, the elongate multicellular bodies have a length ranging from about 1 cm to about 6 m. In some embodiments, the elongate multicellular bodies have a length ranging from about 1 cm to about 5 m. In some embodiments, the elongate multicellular bodies have a length ranging from about 1 cm to about 4 m. In some embodiments, the elongate multicellular bodies have a length ranging from about 1 cm to about 3 m. In some embodiments, the elongate multicellular bodies have a length ranging from about 1 cm to about 2 m. In some embodiments, the elongate multicellular bodies have a length ranging from about 1 cm to about 1 m. In some embodiments, the elongate multicellular bodies have a length ranging from about 1 cm to about 90 cm. In some embodiments, the elongate multicellular bodies have a length ranging from about 1 cm to about 80 cm. In some embodiments, the elongate multicellular bodies have a length ranging from about 1 cm to about 70 cm. In some embodiments, the elongate multicellular bodies have a length ranging from about 1 cm to about 60 cm. In some embodiments, the elongate multicellular bodies have a length ranging from about 1 cm to about 50 cm. In some embodiments, the elongate multicellular bodies have a length ranging from about 1 cm to about 40 cm. In some embodiments, the elongate multicellular bodies have a length ranging from about 1 cm to about 30 cm. In some embodiments, the elongate multicellular bodies have a length ranging from about 1 cm to about 20 cm. In some embodiments, the elongate multicellular bodies have a length ranging from about 1 cm to about 10 cm. In some embodiments, the elongate multicellular bodies have a length ranging from about 2 cm to about 6 cm. In some embodiments, the elongate multicellular bodies have a length of about 1, 2, 3, 4, 5, 6, 7, 8, 9, or 10 mm. In some embodiments, the elongate multicellular bodies have a length of about 1, 2, 3, 4, 5, 6, 7, 8, 9, or 10 cm. In some embodiments, the elongate multicellular bodies have a length of about 1, 2, 3, 4, 5, 6, 7, 8, 9, or 10 m.

In some embodiments, the multicellular bodies have a diameter of about 100, 200, 300, 400, or 500 µm. In some embodiments, the multicellular bodies have a diameter of about 100 µm to about 500 µm. In further embodiments, the multicellular bodies have a diameter of about 200 µm to about 400 µm.

In certain embodiments, the engineered animal skin, hide, or leather provided herein is characterized by a composition that is substantially 60-80 percent aqueous fluid, 14-35 percent protein, and 1-25 percent fat.

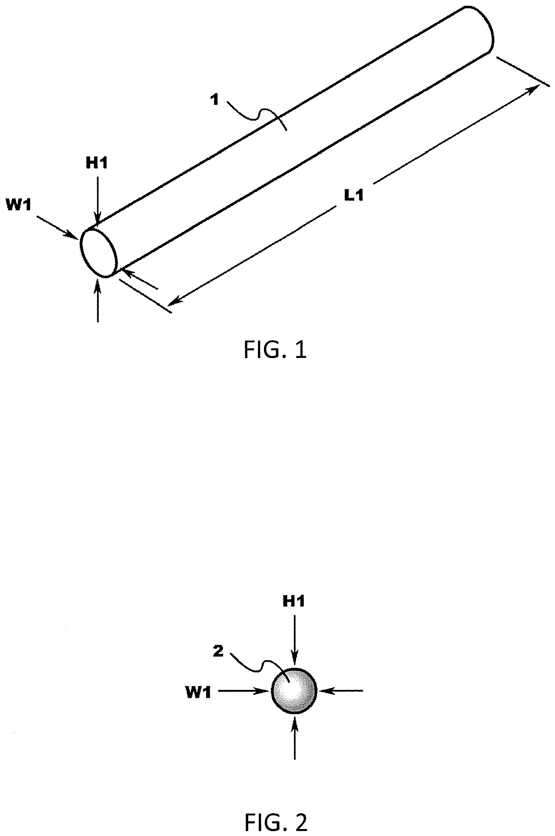

Referring to Fig. 1, in some embodiments, a multicellular body 1 is substantially cylindrical with a width W1 roughly equal to a height HI and has a substantially circular cross section. In further embodiments, a multicellular body 1 is elongate with a length of LI. In still further embodiments, W1 and HI are suitably about 300 to about 600 µm and LI is suitably about 2 cm to about 6 cm.

Referring to Fig. 2, in some embodiments, a multicellular body 2 is substantially spherical with a width W1 roughly equal to a height HI. In further embodiments, W1 and HI are suitably about 300 to about 600 µm.

### Layers

The engineered animal skin, hide, or leather disclosed herein, includes a plurality of layers. In some embodiments, a layer includes a plurality of multicellular bodies comprising a plurality of cultured animal cells wherein the cells are adhered and/or cohered to one another. Also disclosed herein are methods comprising the steps of laying multicellular bodies adjacently onto a support substrate and allowing the multicellular bodies to fuse to form a substantially planar layer for use in formation of engineered animal skin, hide, or leather products. In some embodiments, each layer is biofabricated, using techniques described herein.

In some embodiments, a layer includes homocellular multicellular bodies. In other embodiments, a layer includes heterocellular multicellular bodies. In yet other embodiments, a layer includes both homocellular and heterocellular multicellular bodies. In further embodiments, a layer includes animal epithelial cells, fibroblasts, keratinocytes, corneocytes, melanocytes, Langerhans cells, basal cells, or a combination thereof.

In various embodiments, a layer includes animal fibroblasts and animal keratinocytes in a ratio of about 30:1, 29:1, 28:1, 27:1, 26:1, 25:1, 24:1, 23:1, 22:1, 21:1, 20:1, 19:1, 18:1, 17:1, 16:1, 15:1, 14:1, 13:1, 12:1, 11:1, 10:1, 9:1, 8:1, 7:1, 6:1, 5:1, 4:1, 3:1, 2:1, and 1:1, or increments therein. In some embodiments, a layer contains animal fibroblasts and animal keratinocytes in a ratio of about 20:1 to about 3:1. In various embodiments, a layer includes animal fibroblasts that comprise about 95%, 90%, 85%, 80%, 75%, 70%, 65%, 60%, 55%, 50%, 45%, 40%, 35%, 30%, and 25%, or increments therein, of the total cell population. In some embodiments, a layer includes animal fibroblasts that comprise about 50% to about 95% of the total cell population.

In various embodiments, the thickness of each layer is about 10, 20, 30, 40, 50, 60, 70, 80, 90, 100, 150, 200, 250, 300, 350, 400, 450, 500, 550, 600, 650, 700, 750, 800, 850, 900, 950, 1000, 2000, 3000, 4000, or 5000 urn, or quantifiable increments therein. In some embodiments, the thickness of each layer is chosen to allow diffusion to sufficiently support the maintenance and growth of substantially all the cells in the layer in culture.

In various embodiments, the plurality of layers includes about 2, 3, 4, 5, 6, 7, 8, 9, 10, 15, 20, 25, 30, 35, 40, 45, 50, 55, 60, 65, 70, 75, 80, 85, 90, 95, 100, 150, 200, 250, 300, 350, 400, 450, or 500 layers, or increments therein. In some embodiments, the number of layers is chosen to result in an engineered animal skin, hide, or leather product with thickness that approximates that of a traditional animal skin, hide, or leather product.

In some embodiments, the engineered layers are designed to resemble traditional animal skin, hide, or leather products and design parameters (e.g., cell types, additives, size, shape, etc.) are chosen to approximate those found in traditional animal skin, hide, or leather products. In further embodiments, a layer is characterized by a composition that is substantially similar to traditional animal skin, hide, or leather products. In still further embodiments, a layer is characterized by a composition that is substantially 60-80 percent aqueous fluid, 14-35 percent protein, 1-25 percent fat. In some embodiments, keratinocytes of the engineered layers are aligned. In some embodiments, keratinocytes are aligned by application of an electrical field as is known in the art. In some embodiments, keratinocytes are aligned by application of a mechanical stimulus, such as cyclical stretching and relaxing the substratum, as is known in the art. In further embodiments, aligned (e.g., electro-oriented and mechano-oriented) keratinocytes have substantially the same orientation with regard to each other as is found in many animal skin tissues.

### Additives

In some embodiments, the engineered animal skin, hide, or leather products, engineered layers, and/or multicellular bodies include one or more additives. In further embodiments, one or more additives are selected from: minerals, fiber, fatty acids, and amino acids. In some embodiments, the engineered animal skin, hide, or leather products, layers, and/or multicellular bodies include one or more additives to enhance the commercial appeal (e.g., appearance, color, odor, etc.). In further embodiments, the engineered skin, hide, and leather products, layers, and/or multicellular bodies include one or more colorants, and/or one or more odorants.

In some embodiments, the engineered animal skin, hide, or leather products, engineered layers, and/or multicellular bodies include one or more of: matrix proteins, proteoglycans, antioxidants, perfluorocarbons, and growth factors. The term "growth factor," as used herein, refers to a protein, a polypeptide, or a complex of polypeptides, including cytokines, that are produced by a cell and which can affect itself and/or a variety of other neighboring or distant cells. Typically growth factors affect the growth and/or differentiation of specific types of cells, either developmentally or in response to a multitude of physiological or environmental stimuli. Some, but not all, growth factors are hormones. Exemplary growth factors are insulin, insulin-like growth factor (IGF), nerve growth factor (NGF), vascular endothelial growth factor (VEGF), keratinocyte growth factor (KGF), fibroblast growth factors (FGFs), including basic FGF (bFGF), platelet-derived growth factors (PDGFs), including PDGF-AA and PDGF-AB, hepatocyte growth factor (HGF), transforming growth factor alpha (TGF-a), transforming growth factor beta (TGF-P), including TGFpi and TGFP3, epidermal growth factor (EGF), granulocyte-macrophage colony-stimulating factor (GM-CSF), granulocyte colony-stimulating factor (G-CSF), interleukin-6 (IL-6), IL-8, and the like.

In some embodiments, the engineered animal skin, hide, or leather products, engineered layers, and/or multicellular bodies include one or more preservatives known to the art. In some embodiments, the preservatives are antimicrobial preservatives including, by way of non-limiting examples, calcium propionate, sodium nitrate, sodium nitrite, sulfites (e.g., sulfur dioxide, sodium bisulfite, potassium hydrogen sulfite, etc.) and disodium ethylenediammetetraacetic acid (EDTA). In some embodiments, the preservatives are antioxidant preservatives including, by way of non-limiting examples, butylated hydroxyanisole (BHA) and butylated hydroxytoluene (BHT).

### Support substrate

Disclosed herein, in some embodiments, is a plurality of multicellular bodies arranged adjacently on a support substrate to form a substantially planar layer for use in formation of engineered animal skin, hide, or leather. Also disclosed herein, in some embodiments, are methods comprising arranging multicellular bodies adjacently on a support substrate to form substantially planar layers, laying more than one layer adjacently onto a support substrate, and allowing the layers to fuse to form engineered animal skin, hide, or leather. In further embodiments, each multicellular body and each layer includes animal skin cells. The cells in the central portions of such constructs are typically supplied with oxygen and nutrients by diffusion; however, gasses and nutrients typically diffuse approximately 200-300 microns into three-dimensional cellular constructs.

In some embodiments, the multicellular bodies disclosed herein have a diameter adapted to allow diffusion to sufficiently support the maintenance and growth of said animal skin cells in culture. As a result, in further embodiments, the layers disclosed herein have a thickness adapted to allow diffusion to sufficiently support the maintenance and growth of said animal skin cells in culture.

To facilitate and enhance diffusion, in some embodiments, a support substrate is permeable to fluids, gasses, and nutrients and allows cell culture media to contact all surfaces of multicellular bodies and/or layers during, for example, growth, maturation, and fusion. In various embodiments, a support substrate is made from natural biomaterials, synthetic biomaterials, and combinations thereof. In some embodiments, natural biomaterials include, by way of non-limiting examples, collagen, fibronectin, laminin, and other extracellular matrices. In some embodiments, synthetic biomaterials may include, by way of non-limiting examples, hydroxyapatite, alginate, agarose, polyglycolic acid, polylactic acid, and their copolymers. In some embodiments, a support substrate is solid. In some embodiments, a support substrate is semisolid. In further embodiments, a support substrate is a combination of solid and semisolid support elements.

In some embodiments, the support substrate is raised or elevated above a non-permeable surface, such as a portion of a cell culture environment (e.g., a Petri dish, a cell culture flask, etc.) or a bioreactor. In still further embodiments, an elevated support substrate further facilitates circulation of cell culture media and enhances contact with all surfaces of the multicellular bodies and/or layers.

### Methods of forming multicellular bodies

Also disclosed herein are methods of producing an engineered animal skin, hide, or leather, comprising culturing *in vitro* animal cells comprising one or more types of skin cells, preparing a plurality of elongate or spherical multicellular bodies comprising said animal cells, wherein the cells are cohered to one another, and forming a plurality of planar layers comprising adjacently arranging a plurality of elongate multicellular bodies, wherein said elongate multicellular bodies are fused to form a planar layer.

In some embodiments, methods provided herein further comprise one or more leather processing steps used in traditional leather formation. Examples of processing steps used in traditional leather making include: preserving, soaking, liming, unhairing, fleshing, splitting, deliming, reliming, bating, degreasing, frizing, bleaching, pickling, depickling, tanning, thinning, retanning, lubricating, crusting, wetting, sammying, shaving, rechroming, neutralizing, dyeing, fatliquoring, filling, stripping, stuffing, whitening, fixating, setting, drying, conditioning, milling, staking, buffing, finishing, oiling, brushing, padding, impregnating, spraying, roller coating, curtain coating, polishing, plating, embossing, ironing, glazing, and tumbling. In general, processes that are specific to treating traditional animal hides (e.g., unhairing, fleshing, splitting, etc.) do not need to be performed.

In certain embodiments, said preparing step comprises biofabricating multicellular bodies or said layers. In some embodiments, said preparing step comprises biofabricating multicellular bodies or said layers without a structural scaffold.

In some embodiments, the forming step comprises arranging or placing multicellular bodies on a support substrate that allows the multicellular bodies to fuse to form a substantially planar layer. In some embodiments, said multicellular bodies or said layers are arranged horizontally and/or vertically adjacent to one another.

In some embodiments, said fusing takes place over about 2 hours to about 24 hours.

There are various ways to make multicellular bodies having the characteristics described herein. In some embodiments, a multicellular body can be fabricated from a cell paste containing a plurality of living cells or with a desired cell density and viscosity. In further embodiments, the cell paste can be shaped into a desired shape and a multicellular body formed through maturation (e.g., incubation). In a particular embodiment, an elongate multicellular body is produced by shaping a cell paste including a plurality of living cells into an elongate shape (e.g., a cylinder). In further embodiments, the cell paste is incubated in a controlled environment to allow the cells to adhere and/or cohere to one another to form the elongate multicellular body. In another particular embodiment, a multicellular body is produced by shaping a cell paste including a plurality of living cells in a device that holds the cell paste in a three-dimensional shape. In further embodiments, the cell paste is incubated in a controlled environment while it is held in the three dimensional shape for a sufficient time to produce a body that has sufficient cohesion to support itself on a flat surface, as described herein.

In various embodiments, a cell paste is provided by: (A) mixing cells or cell aggregates (of one or more cell types) and a cell culture medium (e.g., in a pre-determined ratio) to result in a cell suspension, and (B) compacting the cellular suspension to produce a cell paste with a desired cell density and viscosity. In various embodiments, compacting is achieved by a number of methods, such as by concentrating a particular cell suspension that resulted from cell culture to achieve the desired cell concentration (density), viscosity, and consistency required for the cell paste. In a particular embodiment, a relatively dilute cell suspension from cell culture is centrifuged for a determined time to achieve a cell concentration in the pellet that allows shaping in a mold. Tangential flow filtration ("TFF") is another suitable method of concentrating or compacting the cells. In some embodiments, compounds are combined with the cell suspension to lend the extrusion properties required. Suitable compounds include, by way of non-limiting examples, collagen, hydrogels, Matrigel, nanofibers, self-assembling nanofibers, gelatin, fibrinogen, etc.

In some embodiments, the cell paste is produced by mixing a plurality of living cells with a tissue culture medium, and compacting the living cells (e.g., by centrifugation). One or more ECM component (or derivative of an ECM component) is optionally included by, resuspending the cell pellet in one or more physiologically acceptable buffers containing the ECM component(s) (or derivative(s) of ECM component(s)) and the resulting cell suspension centrifuged again to form the cell paste.

In some embodiments, the cell density of the cell paste desired for further processing may vary with cell types. In further embodiments, interactions between cells determine the properties of the cell paste, and different cell types will have a different relationship between cell density and cell-cell interaction. In still further embodiments, the cells may be pre-treated to increase cellular interactions before shaping the cell paste. For example, cells may be incubated inside a centrifuge tube after centrifugation in order to enhance cell-cell interactions prior to shaping the cell paste.

In various embodiments, many methods are used to shape the cell paste. For example, in a particular embodiment, the cell paste is manually molded or pressed (e.g., after concentration/compaction) to achieve a desired shape. By way of a further example, the cell paste may be taken up (e.g., aspirated) into a preformed instrument, such as a micropipette (e.g., a capillary pipette), that shapes the cell paste to conform to an interior surface of the instrument. The cross sectional shape of the micropipette (e.g., capillary pipette) is alternatively circular, square, rectangular, triangular, or other non-circular cross-sectional shape. In some embodiments, the cell paste is shaped by depositing it into a preformed mold, such as a plastic mold, metal mold, or a gel mold. In some embodiments, centrifugal casting or continuous casting is used to shape the cell paste.

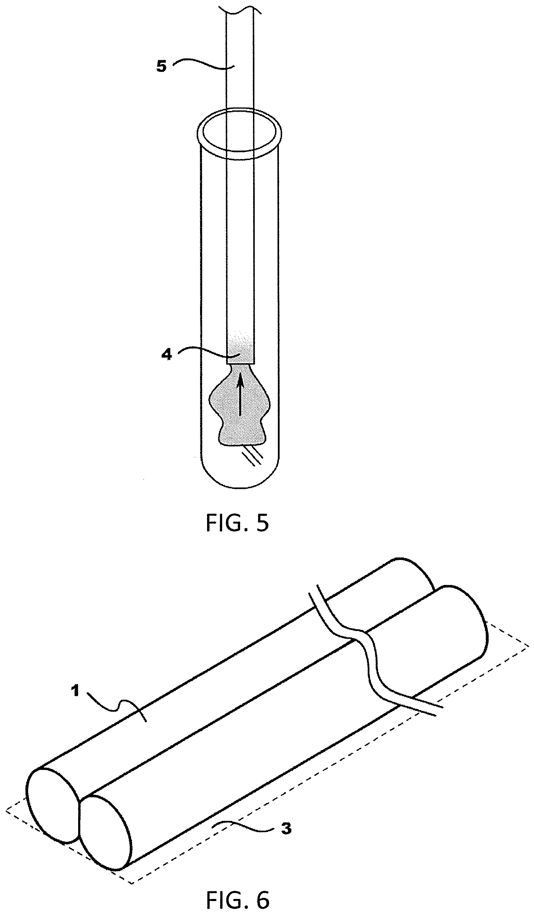

Referring to Fig. 5, in a particular example, the shaping includes retaining the cell paste 4 in a shaping device 5 (e.g., a capillary pipette) to allow the cells to partially adhere and/or cohere to one another in the shaping device. By way of further example, cell paste can be aspirated into a shaping device and held in the shaping device for a maturation period (also referred to herein as an incubation period) to allow the cells to at least partially adhere and/or cohere to one another. In some embodiments, the shaping device (e.g., capillary pipette) is part of a printing head of an apparatus operable to automatically place the multicellular body in a three-dimensional construct. However, there is a limit to the amount of time cells can remain in a shaping device such as a capillary pipette, which provides the cells only limited access at best to oxygen and/or nutrients, before viability of the cells is impacted.

In some embodiments, a partially adhered and/or cohered cell paste is transferred from the shaping device (e.g., capillary pipette) to a second shaping device (e.g., a mold) that allows nutrients and/or oxygen to be supplied to the cells while they are retained in the second shaping device for an additional maturation period. One example of a suitable shaping device that allows the cells to be supplied with nutrients and oxygen is a mold for producing a plurality of multicellular bodies (e.g., substantially identical multicellular bodies). By way of further example, such a mold includes a biocompatible substrate made of a material that is resistant to migration and ingrowth of cells into the substrate and resistant to adherence of cells to the substrate. In various embodiments, the substrate can suitably be made of Teflon®, (PTFE), stainless steel, agarose, polyethylene glycol, glass, metal, plastic, or gel materials (e.g., agarose gel or other hydrogel), and similar materials. In some embodiments, the mold is also suitably configured to allow supplying tissue culture media to the cell paste (e.g., by dispensing tissue culture media onto the top of the mold).

In a particular embodiment, a plurality of elongate grooves are formed in the substrate. In a further particular embodiment, the depth of each groove is in the range of about 500 microns to about 1000 microns and the bottom of each groove has a semicircular cross-sectional shape for forming elongate cylindrical multicellular bodies that have a substantially circular cross-sectional shape. In a further particular embodiment, the width of the grooves is suitably slightly larger than the width of the multicellular body to be produced in the mold. For example, the width of the grooves is suitably in the range of about 300 microns to about 1000 microns.

Thus, in embodiments where a second shaping device is used, the partially adhered and/or cohered cell paste is transferred from the first shaping device (e.g., a capillary pipette) to the second shaping device (e.g., a mold). In further embodiments, the partially adhered and/or cohered cell paste can be transferred by the first shaping device (e.g., the capillary pipette) into the grooves of a mold. In still further embodiments, following a maturation period in which the mold is incubated along with the cell paste retained therein in a controlled environment to allow the cells in the cell paste to further adhere and/or cohere to one another to form the multicellular body, the cohesion of the cells will be sufficiently strong to allow the resulting multicellular body to be picked up with an implement (e.g., a capillary pipette). In still further embodiments, the capillary pipette is suitably part of a printing head of an apparatus operable to automatically place the multicellular body into a three-dimensional construct.

In some embodiments, the cross-sectional shape and size of the multicellular bodies will substantially correspond to the cross-sectional shapes and sizes of the first shaping device and optionally the second shaping device used to make the multicellular bodies, and the skilled artisan will be able to select suitable shaping devices having suitable cross-sectional shapes, cross-sectional areas, diameters, and lengths suitable for creating multicellular bodies having the cross-sectional shapes, cross-sectional areas, diameters, and lengths discussed above.

As discussed herein, a large variety of cell types may be used to create the multicellular bodies of the present invention. Thus, one or more types of cells or cell aggregates including, for example, all of the cell types listed herein, may be employed as the starting materials to create the cell paste. For instance, cells such as animal epithelial cells, fibroblasts, keratinocytes, corneocytes, melanocytes, Langerhans cells, basal cells, or a combination thereof are optionally employed. As described herein, a multicellular body is homocellular or heterocellular. For making homocellular multicellular bodies, the cell paste suitably is homocellular, i.e., it includes a plurality of living cells of a single cell type. For making heterocellular multicellular bodies, on the other hand, the cell paste will suitably include significant numbers of cells of more than one cell type (i.e., the cell paste will be heterocellular). As described herein, when heterocellular cell paste is used to create the multicellular bodies, the living cells may "sort out" during the maturation and cohesion process based on differences in the adhesive strengths of the cells, and may recover their physiological conformation.

In some embodiments, in addition to the plurality of living cells, one or more ECM components or one or more derivatives of one or more ECM components (e.g., gelatin, fibrinogen, collagen, fibronectin, laminin, elastin, and/or proteoglycans) can suitably be included in the cell paste to incorporate these substances into the multicellular bodies, as noted herein. In further embodiments, adding ECM components or derivatives of ECM components to the cell paste may promote cohesion of the cells in the multicellular body. For example, gelatin and/or fibrinogen are optionally added to the cell paste. More particularly, a solution of 10-30% gelatin and a solution of 10-80 mg/ml fibrinogen are optionally mixed with a plurality of living cells to form a cell suspension containing gelatin and fibrinogen.

Various methods are suitable to facilitate the further maturation process. In one embodiment, the cell paste may be incubated at about 37°C for a time period (which may be cell-type dependent) to foster adherence and/or coherence. Alternatively or in addition, the cell paste may be held in the presence of cell culture medium containing factors and/or ions to foster adherence and/or coherence.

### Arranging multicellular bodies on a support substrate to form layers

A number of methods are suitable to arrange multicellular bodies on a support substrate to produce a desired three-dimensional structure (e.g., a substantially planar layer). For example, in some embodiments, the multicellular bodies are manually placed in contact with one another, deposited in place by extrusion from a pipette, nozzle, or needle, or positioned in contact by an automated machine such as a biofabricator.

As described herein, in some embodiments, the support substrate is permeable to fluids, gasses, and nutrients and allows cell culture media to contact all surfaces of the multicellular bodies and/or layers during arrangement and subsequent fusion. As further described herein, in some embodiments, a support substrate is made from natural biomaterials such as collagen, fibronectin, laminin, and other extracellular matrices. In some embodiments, a support substrate is made from synthetic biomaterials such as hydroxyapatite, alginate, agarose, polyglycolic acid, polylactic acid, and their copolymers. In some embodiments, a support substrate is solid. In some embodiments, a support substrate is semisolid. In further embodiments, a support substrate is a combination of solid and semisolid support elements. In further embodiments, a support substrate is planar to facilitate production of planar layers. In some embodiments, the support substrate is raised or elevated above a non-permeable surface, such as a portion of a cell culture environment (e.g., a Petri dish, a cell culture flask, etc.) or a bioreactor. Therefore, in some embodiments, a permeable, elevated support substrate contributes to prevention of premature cell death, contributes to enhancement of cell growth, and facilitates fusion of multicellular bodies to form layers.

As described herein, in various embodiments, multicellular bodies have many shapes and sizes. In some embodiments, multicellular bodies are elongate and in the shape of a cylinder. *See* e.g., Figs. 1 and 3. In some embodiments, elongate multicellular bodies are of similar lengths and/or diameters. In other embodiments, elongate multicellular bodies are of differing lengths and/or diameters. In some embodiments, multicellular bodies are substantially spherical. *See* e.g., Figs. 2 and 4. In some embodiments, layers include substantially spherical multicellular bodies that are substantially similar in size. In other embodiments, layers include substantially spherical multicellular bodies that are of differing sizes.

Referring to Fig. 6, in some embodiments, elongate multicellular bodies 1 are arranged on a support substrate 3 horizontally adjacent to, and in contact with, one or more other elongate multicellular bodies to form a substantially planar layer.

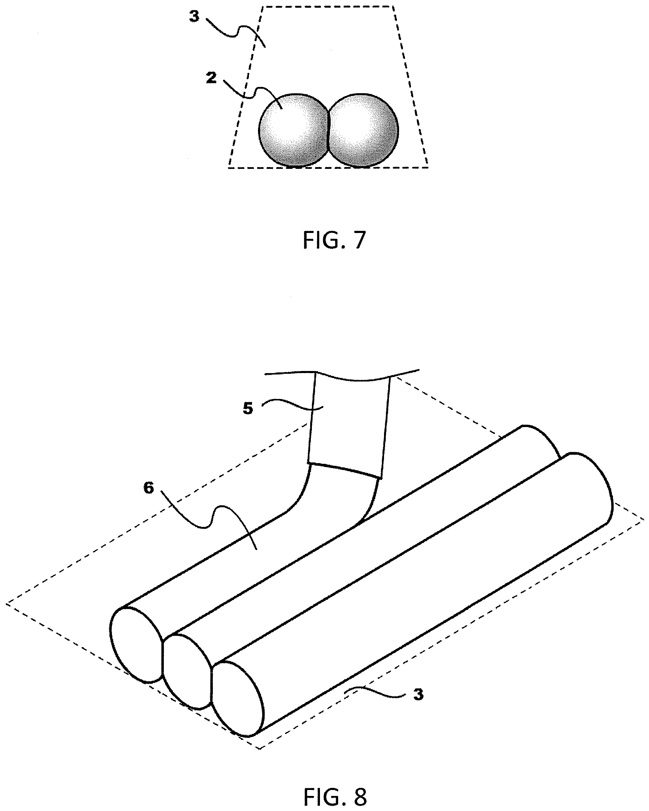

Referring to Fig. 7, in some embodiments, substantially spherical multicellular bodies 2 are arranged on a support substrate 3 horizontally adjacent to, and in contact with, one or more other substantially spherical multicellular bodies. In further embodiments, this process is repeated to build up a pattern of substantially spherical multicellular bodies, such as a grid, to form a substantially planar layer.

Referring to Fig. 8, in a particular embodiment, an elongate multicellular 6 body is laid onto a support substrate 3 via an implement such as a capillary pipette 5 such that it is horizontally adjacent to, and in contact with one or more other multicellular bodies. In further embodiments, an elongate multicellular body is laid onto a support substrate such that it is parallel with a plurality of other elongate multicellular bodies.

Referring to Fig. 9, in some embodiments, a subsequent series of elongate multicellular bodies 8 are arranged vertically adjacent to, and in contact with, a prior series of elongate multicellular bodies 9 on a support substrate 3 to form a thicker layer.

In other embodiments, layers of different shapes and sizes are formed by arranging multicellular bodies of various shapes and sizes. In some embodiments, multicellular bodies of various shapes, sizes, densities, cellular compositions, and/or additive compositions are combined in a layer and contribute to, for example, appearance, taste, and texture of the resulting layer.

Referring to Fig. 10, in some embodiments, elongate multicellular bodies 9 are arranged adjacent to, and in contact with, substantially spherical multicellular bodies 10 on a support substrate 3 to form a complex layer.

Once assembly of a layer is complete, in some embodiments, a tissue culture medium is poured over the top of the construct. In further embodiments, the tissue culture medium enters the spaces between the multicellular bodies to support the cells in the multicellular bodies. The multicellular bodies in the three-dimensional construct are allowed to fuse to one another to produce a substantially planar layer for use in formation of engineered animal skin, hide, and leather. By "fuse," "fused" or "fusion," it is meant that the cells of contiguous multicellular bodies become adhered and/or cohered to one another, either directly through interactions between cell surface proteins, or indirectly through interactions of the cells with ECM components or derivatives of ECM components. In some embodiments, a fused layer is completely fused and that multicellular bodies have become substantially contiguous. In some embodiments, a fused layer is substantially fused or partially fused and the cells of the multicellular bodies have become adhered and/or cohered to the extent necessary to allow moving and manipulating the layer intact.

In some embodiments, the multicellular bodies fuse to form a layer in a cell culture environment (e.g., a Petri dish, cell culture flask, bioreactor, etc.). In further embodiments, the multicellular bodies fuse to form a layer in an environment with conditions suitable to facilitate growth of the cell types included in the multicellular bodies. In various embodiments, fusing takes place over about 15, 20, 25, 30, 35, 40, 45, 50, 55, and 60 minutes, and increments therein. In other various embodiments, fusing takes place over about 1, 2, 3, 4, 5, 6, 7, 8, 9, 10, 11, 12, 14, 16, 18, 20, 22, 24, 26, 28, 30, 32, 34, 36, 38, 40, 42, 44, 46, and 48 hours, and increments therein. In yet other various embodiments, fusing takes place over about 1, 2, 3, 4, 5, 6, 7, 8, 9, 10, 12, and 14 days, and increments therein. In further embodiments, fusing takes place over about 2 hours to about 24 hours. Several factors influence the fusing time required including, by way of non-limiting examples, cell types, cell type ratios, culture conditions, and the presence of additives such as growth factors.

Once fusion of a layer is complete, in some embodiments, the layer and the support substrate are separated. In other embodiments, the layer and the support substrate are separated when fusion of a layer is substantially complete or partially complete, but the cells of the layer are adhered and/or cohered to one another to the extent necessary to allow moving, manipulating, and stacking the layer without breaking it apart. In further embodiments, the layer and the support substrate are separated via standard procedures for melting, dissolving, or degrading the support substrate. In still further embodiments, the support substrate is dissolved, for example, by temperature change, light, or other stimuli that do not adversely affect the layer. In a particular embodiment, the support substrate is made of a flexible material and peeled away from the layer.

In some embodiments, the separated layer is transferred to a bioreactor for further maturation. In some embodiments, the separated layer matures and further fuses after incorporation into an engineered animal skin, hide, or leather product.

In other embodiments, the layer and the support substrate are not separated. In further embodiments, the support substrate degrades or biodegrades prior to packaging, freezing, sale or consumption of the assembled engineered animal skin, hide, or leather product.

### Arranging layers on a support substrate to form animal skin, hide, or leather

A number of methods are suitable to arrange layers on a support substrate to produce engineered animal skin, hide, or leather. For example, in some embodiments, the layers are manually placed in contact with one another or deposited in place by an automated, computer-aided machine such as a biofabricator, according to a computer script. In further embodiments, substantially planar layers are stacked to form engineered animal skin, hide, or leather.

In various embodiments, about 2, 3, 4, 5, 6, 7, 8, 9, 10, 15, 20, 25, 30, 35, 40, 45, or 50 layers, or increments therein, are stacked. In various embodiments, about 55, 60, 65, 70, 75, 80, 85, 90, 95, 100, 150, 200, 250, 300, 350, 400, 450, or 500 layers, or increments therein, are stacked. In some embodiments, about 10 to about 100 layers are stacked. In some embodiments, about 10 to about 90 layers are stacked. In some embodiments, about 10 to about 80 layers are stacked. In some embodiments, about 10 to about 70 layers are stacked. In some embodiments, about 10 to about 60 layers are stacked. In some embodiments, about 10 to about 50 layers are stacked. In some embodiments, about 20 to about 80 layers are stacked. In some embodiments, about 20 to about 70 layers are stacked. In some embodiments, about 20 to about 60 layers are stacked. In some embodiments, about 20 to about 50 layers are stacked. In some embodiments, about 20 to about 40 layers are stacked. In some embodiments, about 20 to about 30 layers are stacked. In some embodiments, about 40 to about 60 layers are stacked. In further embodiments, stacking is repeated to develop a thickness that approximates a traditional animal skin, hide, or leather product. In various embodiments, stacked layers comprise an engineered animal skin, hide, or leather product about 1, 2, 3, 4, 5, 6, 7, 8, 9, or 10 mm, or increments therein, thick.

In some embodiments, a layer has an orientation defined by the placement, pattern, or orientation of multicellular bodies. In further embodiments, each layer is stacked with a particular orientation relative to the support substrate and/or one or more other layers. In various embodiments, one or more layers is stacked with an orientation that includes rotation relative to the support substrate and/or the layer below, wherein the rotation is about 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, and 180 degrees, or increments therein. In other embodiments, all layers are oriented substantially similarly.

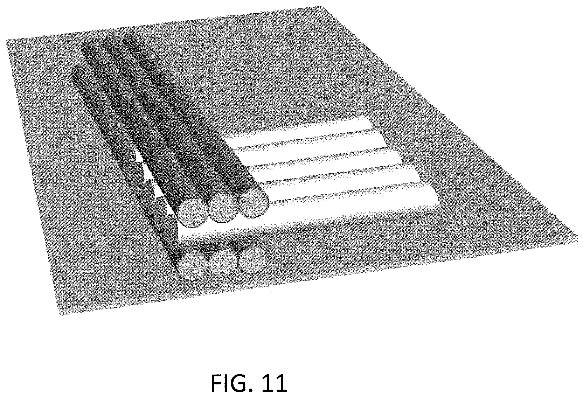

Referring to Fig. 11, in a particular embodiment, layers have an orientation defined by the parallel placement of elongate multicellular bodies used to form the layer. In a further particular embodiment, layers are stacked with an orientation including 90 degree rotation with respect to the layer below to form engineered animal skin, hide, or leather.

Once stacking of the layers is complete, in some embodiments, the layers in the three-dimensional construct are allowed to fuse to one another to produce engineered animal skin, hide, or leather. In some embodiments, the layers fuse to form engineered animal skin, hide, or leather in a cell culture environment (e.g., a Petri dish, cell culture flask, bioreactor, etc.). In various embodiments, fusing takes place over about 15, 20, 25, 30, 35, 40, 45, 50, 55, and 60 minutes, and increments therein. In other various embodiments, fusing takes place over about 1, 2, 3, 4, 5, 6, 7, 8, 9, 10, 11, 12, 14, 16, 18, 20, 22, 24, 26, 28, 30, 32, 34, 36, 38, 40, 42, 44, 46, and 48 hours, and increments therein. In further embodiments, fusing takes place over about 2 hours to about 24 hours.

In some embodiments, once stacked, the cells of the multicellular bodies and layers begin to die due to the inability of gases, fluids, and nutrients, to diffuse into or otherwise reach the inner portions of the construct. In further embodiments, the gradual death of the cells is similar to the natural cell death that occurs in the tissues of a postmortem organism. In some embodiments, the layers of the engineered animal skin, hide, or leather construct fuse to one another simultaneously with the gradual death of the cells. In some embodiments, the multicellular bodies of the layers continue to fuse to one another simultaneously with the gradual death of the cells. In further embodiments, fusion within and between layers is complete or substantially complete prior to the death of a majority of the cells of the construct. In further embodiments, fusion within and between layers is complete or substantially complete prior to the death of all the cells of the construct.

Once assembly of the engineered animal skin, hide, or leather is complete, in some embodiments, the animal skin, hide, or leather and the support substrate are separated. In further embodiments, the animal skin, hide, or leather and the support substrate are separated via standard procedures for melting, dissolving, or degrading the support substrate. In still further embodiments, the support substrate is dissolved, for example, by temperature change, light, or other stimuli that do not adversely affect the animal skin, hide, or leather. In a particular embodiment, the support substrate is made of a flexible material and peeled away from the animal skin, hide, or leather. In some embodiments, the separated animal skin, hide, or leather is transferred to a bioreactor for further maturation. In other embodiments, the animal skin, hide, or leather and the support substrate are not separated. In further embodiments, the support substrate degrades or biodegrades prior to sale or consumption.

In some embodiments, the animal skin, hide, or leather is irradiated. In some embodiments, the animal skin, hide, or leather is processed to prevent decomposition or degradation prior to distribution and sale.

### Engineered animal skin, hide, and leather

Disclosed herein, in some embodiments, is engineered animal skin, hide, or leather products. Also disclosed herein, in various embodiments, is a plurality of multicellular bodies arranged adjacently on a support substrate to form a substantially planar layer for use in formation of engineered animal skin, hide, or leather.

In some embodiments, the engineered animal skin, hide, or leather products are further processed by any known methods in the art. Examples of known methods of processing include processing by preserving, soaking, liming, unhairing, fleshing, splitting, deliming, reliming, bating, degreasing, frizing, bleaching, pickling, depickling, tanning, thinning, retanning, lubricating, crusting, wetting, sammying, shaving, rechroming, neutralizing, dyeing, fatliquoring, filling, stripping, stuffing, whitening, fixating, setting, drying, conditioning, milling, staking, buffing, finishing, oiling, brushing, padding, impregnating, spraying, roller coating, curtain coating, polishing, plating, embossing, ironing, glazing, and tumbling. It is significant that the methods described herein do not require any of the pre-processing steps that are necessary when using natural animal hide, including de-hairing (unhairing), liming, fleshing, splitting deliming, reliming, etc. The layered bodies formed as described herein may be formed of any appropriate length, and the collagen (and other ECM molecules) formed by the cultured cells, resulting in a layered body that does not include structures such as hair follicles, blood vessels, muscle (e.g., arrector pili muscle), etc.

In general, engineered leather described herein may be tanned (or processed by a similar process) to modify the extracellular matrix material. As discussed above, one of the principle components of the ECM is collagen (and particularly Type I collagen). Tanning may modify the collagen. For example, one tanning agent, chromium(III) sulfate ([Cr(H2O)6]2(SO4)3), has long been regarded as the most efficient and effective tanning agent. Chromium(III) sulfate dissolves to give the hexaaquachromium(III) cation, [Cr(H2O)6]3+, which at higher pH undergoes processes called olation to give polychromium(III) compounds that are active in tanning, being the cross-linking of the collagen subunits. Some ligands include the sulfate anion, the collagen's carboxyl groups, amine groups from the side chains of the amino acids, as well as masking agents. Masking agents are carboxylic acids, such as acetic acid, used to suppress formation of polychromium(III) chains. Masking agents allow the tanner to further increase the pH to increase collagen's reactivity without inhibiting the penetration of the chromium(III) complexes. Collagen's high content of hydroxyproline allows for significant cross-linking by hydrogen bonding within the helical structure. Ionized carboxyl groups (RCO2-) are formed by hydrolysis of the collagen by the action of hydroxide. This conversion may occur during the liming process, before introduction of the tanning agent (chromium salts). The ionized carboxyl groups may coordinate as ligands to the chromium(III) centers of the oxo-hydroxide clusters. Tanning may increase the spacing between protein chains in collagen (e.g., from 10 to 17 Å), consistent with cross-linking by polychromium species, of the sort arising from olation and oxolation. The chromium may be cross-linked to the collagen. Chromium's ability to form such stable bridged bonds explains why it is considered one of the most efficient tanning compounds. Chromium-tanned leather can contain between 4 and 5% of chromium. This efficiency is characterized by its increased hydrothermal stability of the leather, and its resistance to shrinkage in heated water. Other tanning agents may be used to tan the layered body and modify the collagen.

In some embodiments, the engineered animal skin, hide, or leather products are substantially-free of pathogenic microorganisms. In further embodiments, controlled and substantially sterile methods of cell preparation, cell culture, multicellular body preparation, layer preparation, and engineered animal skin, hide, or leather preparation result in a product substantially-free of pathogenic microorganisms. In further embodiments, an additional advantage of such a product is increased utility and safety.

In some embodiments, the engineered animal skin, hide, or leather products are shaped. In further embodiments, the animal skin, hide, or leather is shaped by, for example, controlling the number, size, and arrangement of the multicellular bodies and/or the layers used to construct the animal skin, hide, or leather. In other embodiments, the animal skin, hide, or leather is shaped by, for example, cutting, pressing, molding, or stamping. In some embodiments, the shape of the animal skin, hide, or leather product is selected to resemble a traditional animal skin, hide, or leather product.

### Examples

The following illustrative examples are representative of embodiments of the methods of forming bodies that can be tanned to form engineered leather. The examples described herein and are not meant to be limiting.

### Example 1 - Preparation of support substrate

To prepare a 2% agarose solution, 2 g of Ultrapure Low Melting Point (LMP) agarose was dissolved in 100 mL of ultrapure water/buffer solution (1:1, v/v). The buffer solution is optionally PBS (Dulbecco's phosphate buffered saline Ix) or HBSS (Hanks' balanced salt solution lx). The agarose solution was placed in a beaker containing warm water (over 80°C) and held on the hot plate until the agarose dissolves completely. The agarose solution remains liquid as long as the temperature is above 36°C. Below 36°C, a phase transition occurs, the viscosity increases, and finally the agarose forms a gel.

To prepare agarose support substrate, 10 mL of liquid 2% agarose (temperature >40°C) was deposited in a 10 cm diameter Petri dish and evenly spread to form a uniform layer. Agarose was allowed for form a gel at 4°C in a refrigerator.

### Example 2 - Culture of bovine keratinocvtes, fibroblasts, and epithelial cells

Freshly isolated bovine keratinocytes, fibroblasts, and epithelial cells were grown in low glucose DMEM with 10% fetal bovine serum (Hyclone Laboratories, UT), 10% porcine serum (Invitrogen), L-ascorbic acid, copper sulfate, HEPES, L-proline, L-alanine, L-glycine, and Penicillin G (all aforementioned supplements were purchased from Sigma, St. Louis, MO). Cell lines were cultured on 0.5%> gelatin (porcine skin gelatin; Sigma) coated dishes (Techno Plastic Products, St. Louis, MO) and were maintained at 37°C in a humidified atmosphere containing 5% CO2. The keratinocytes were subcultured up to passage 7 before being used to form multicellular bodies.

### Example 3 - Preparation of multicellular spheroids and cylinders

Cell cultures were washed twice with phosphate buffered saline solution (PBS, Invitrogen) and treated for 10 min with 0.1% Trypsin (Invitrogen) and centrifuged at 1500 RPM for 5 min. Cells were resuspended in 4 mL of cell-type specific medium and incubated in 10-mL tissue culture flasks (Bellco Glass, Vineland, NJ) at 37°C with 5% CO2 on gyratory shaker (New Brunswick Scientific, Edison, NJ) for one hour, for adhesion recovery and centrifuged at 3500 RPM. The resulting pellets were transferred into capillary micropipettes of 300 µm (Sutter Instrument, CA) or 500 µm (Drummond Scientific Company, Broomall, PA) diameters and incubated at 37°C with 5% CO2 for 15 min. For spherical multicellular bodies, extruded cylinders were cut into equal fragments that were let to round up overnight on a gyratory shaker. Depending on the diameter of the micropipettes, this procedure provided regular spheroids of defined size and cell number. For cylindrical multicellular bodies, cylinders were mechanically extruded into specifically prepared non-adhesive Teflon® or agarose molds using a biofabricator. After overnight maturation in the mold, cellular cylinders were cohesive enough to be deposited.

The multicellular bodies were packaged into cartridges (micropipettes of 300-500 µm inner diameter). Cartridges were inserted into a biofabricator and delivered onto a support substrate according to a computer script that encodes the shape of the structure to be fabricated.

### Example 4 - Preparation of engineered animal skin, hide, and leather

Cylindrical multicellular bodies are prepared as described in Example 3. The multicellular bodies are heterocellular and composed of the bovine keratinocytes, dermal fibroblasts, and epithelial cells of Example 2. The ratio of keratinocytes to dermal fibroblasts in the multicellular bodies is about 19:1. The multicellular bodies have a cross-sectional diameter of 300 µm and a length of either 2 cm, 3 cm, 4 cm, or 5 cm. Matured and multicellular bodies are packaged into cartridges (micropipettes of 300 µm inner diameter), which are then inserted into a biofabricator.

An agarose support substrate is prepared as described in Example 1. The support substrate is raised above the bottom of a large Petri dish by a fine mesh pedestal such that cell culture media may contact all surfaces of the multicellular bodies and layers deposited onto the substrate.

A biofabricator delivers the multicellular bodies onto the support substrate according to the instructions of a computer script. The script encodes placement of cylindrical multicellular bodies to form a substantially square mono layer with an average width of about 10 cm and an average length of about 10 cm. The multicellular bodies are placed parallel to one another with bodies of varying lengths placed end to end to form the encoded shape.

Culture medium is poured over the top of the layer and the construct is allowed to partially fuse over the course of about 12 hours at 37°C in a humidified atmosphere containing 5% CO2. During this time, the cells of the multicellular bodies adhere and/or cohere to the extent necessary to allow moving and manipulating the layer without breaking it apart.

The partially fused layers are peeled from the support and stacked. Sixty-five layers are stacked to form the engineered animal skin, hide, or leather, which has an overall width and height of about 2 cm and a length and width of about 10 cm. Each layer is rotated 90 degrees with respect to the layer below. Once stacked, the cells start dying due to oxygen deprivation, as culture medium is not changed. Cell death starts in the stack's interior, as these are the first deprived of oxygen, and progressively reaches outer cells, as the surrounding culture medium gets gradually depleted in oxygen. Simultaneously with cell death the partially fused layers continue to fuse while they start fusing also in the vertical direction. Since the fusion process takes about 6 hours, while cell death takes about 20 hours, the postmortem construct is fully fused and assumes a shape similar to a traditional animal skin, hide, or leather. The animal skin, hide, or leather is further processed by traditional preparation, tanning, and/or crusting methods.

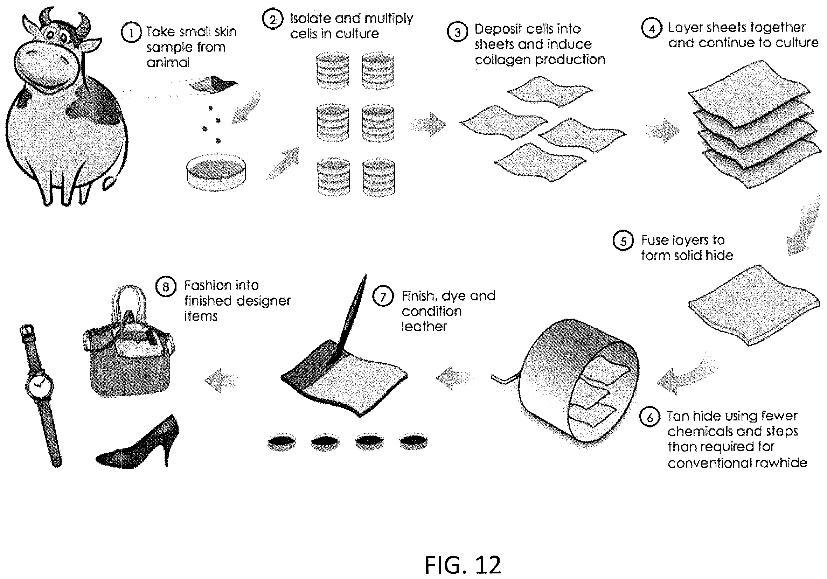

FIG. 12 shows a high-level overview of a method of forming engineered leather as described herein. FIG. 12 shows 8 "steps" illustrating the formation of artificial leather for use in commercial goods. As discussed and illustrated above, initially, skin cells are cultured from an appropriate source. In FIG. 12 (1), the source is a skin sample from an animal (shown as a cow). The cells are cultured *in vitro* and expanded, as shown in step 12 (2); these cells may be used to form multicellular bodies, as discussed above. Cultured skin cells may then be deposited to form sheet or layers, as illustrated in step 12 (3). The cells may be deposited as multicellular bodies (tubes, spheres, etc.) and allowed to fuse and form (or form additional) extracellular matrix (ECM), typically including collagen. Cells typically release ECM including collagen when cultured in monolayers, as known in the art. In some variations, collagen synthesis and/or release may be induced with additional agents and/or by washing to remove inhibitors of collagen release.

The sheets may then be layered together, as illustrated generally in FIG. 12 (4). The layers may be stacked onto one another individually (e.g., by sequential additional of layers), or concurrently (e.g., forming layers of two layers, then combining the deal layers, then the quadruple layers, etc., or by stacking them all together at once, etc.). In some variations the layers are stacked sequentially. The layers may be treated before stacking.

After an appropriate time, the layers are allowed to fuse to form a single body, which may be referred to a layered body as shown in FIG. 12 (5). Fusion may occur by the activity of skin cells within one (or more) of the stacked layers continuing to form and release ECM. The multicellular bodies may fuse as discussed above; even after fusion between the layers, the pattern of the ECM (e.g., collagen) within each layer may reflect the fabrication method used. For example, a section through the fused layered body may still reflect strata reflecting the layered nature of the formation process.

The layered body may then be tanned, as shown in FIG. 12 (6). In the example shown in FIG. 12, multiple layered bodies may be tanned together, and the leather formed may be post-processed to finish, including dying and conditioning, as shown in FIG. 12 (7). Finally, the leather may be used to form objects which would otherwise use traditional ("natural") leather, as shown in FIG. 12 (8).

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

As mentioned above, terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements, these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/-5% of the stated value (or range of values), +/- 10% of the stated value (or range of values), etc. Any numerical range recited herein is intended to include all sub-ranges subsumed therein.

Although various illustrative embodiments are described above, any of a number of changes may be made to various embodiments without departing from the scope of the invention as described by the claims. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the invention as it is set forth in the claims.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, if more than one is, in fact, disclosed. Thus, although specific embodiments have been illustrated and described herein, any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description.

## Claims

1. A method of producing an engineered leather, the method comprising:
culturing one or more types of collagen-producing cells *in vitro*;
forming a plurality of sheets of extracellular matrix including collagen produced by the one or more types of collagen-producing cells;
layering the plurality of sheets to form a body having a volume; and
processing the body by tanning;
wherein forming the plurality of sheets comprises forming a plurality of planar layers comprising adjacently arranging a plurality of elongate multicellular bodies, wherein said elongate multicellular bodies are fused to form a planar layer.

2. The method of claim 1, wherein arranging comprises placing multicellular bodies on a support substrate that allows the multicellular bodies to fuse to form a substantially planar layer;
optionally wherein the support substrate is permeable to fluids and nutrients to allow cell culture media to contact all surfaces of said layer.

3. The method of claim 1, wherein:
forming comprises automated deposition of multicellular bodies into said layers without a structural scaffold; or
said multicellular bodies are arranged horizontally and/or vertically adjacent to one another; or
said fusing takes place over 2 hours to 24 hours.

4. The method of claim 1, wherein said elongate multicellular bodies:
are of collagen-producing cells and are of the same lengths; or
have a length ranging from 1 mm to 10 m; or
have a length ranging from 1 cm to 1 m; or
have a length ranging from 1 cm to 50 cm.

5. The method of claim 1, further comprising processing the body using one or more additional processing steps, optionally wherein the additional processing step is selected from the group consisting of preserving, soaking, bating, pickling, depickling, thinning, retanning, lubricating, crusting, wetting, sammying, shaving, rechroming, neutralizing, dyeing, fatliquoring, filling, stripping, stuffing, whitening, fixating, setting, drying, conditioning, milling, staking, buffing, finishing, oiling, brushing, padding, impregnating, spraying, roller coating, curtain coating, polishing, plating, embossing, ironing, glazing, and tumbling.

6. The method of claim 1, wherein:
said collagen-producing cells comprise one or more of: epithelial cells, fibroblasts, keratinocytes, corneocytes, melanocytes, Langerhans cells, basal cells, or a combination thereof; or
said epithelial cells comprise squamous cells, cuboidal cells, columnar cells, basal cells, or a combination thereof; or
said fibroblasts are dermal fibroblasts; or
said keratinocytes are epithelial keratinocytes, basal keratinocytes, proliferating basal keratinocytes, differentiated suprabasal keratinocytes, or a combination thereof; or
said collagen-producing cells are substantially free of non-differentiated keratinocytes, fibroblasts, or epithelial cells.

7. The method of claim 1, wherein forming the plurality of sheets comprises forming a plurality of sheets of the one or more types of collagen-producing cells and extracellular matrix material including collagen and one or more components selected from the group consisting of: keratin, elastin, gelatin, proteoglycan, dermatan sulfate proteoglycan, glycosoaminoglycan, fibronectin, laminin, dermatopontin, lipid, fatty acid, carbohydrate, and a combination thereof.

8. The method of claim 1, wherein said collagen-producing cells are derived from:
mammals selected from the group consisting of antelope, bear, beaver, bison, boar, camel, caribou, cat, cattle, deer, dog, elephant, elk, fox, giraffe, goat, hare, horse, ibex, kangaroo, lion, llama, lynx, mink, moose, oxen, peccary, pig, rabbit, seal, sheep, squirrel, tiger, whale, wolf, yak, and zebra, and a combination thereof; or
reptiles selected from the group consisting of turtle, snake, crocodile, and alligator, or combinations thereof; or
birds selected from the group consisting of chicken, duck, emu, goose, grouse, ostrich, pheasant, pigeon, quail, and turkey, or combinations thereof; or
fish selected from the group consisting of anchovy, bass, catfish, carp, cod, eel, flounder, fugu, grouper, haddock, halibut, herring, mackerel, mahi mahi, manta ray, marlin, orange roughy, perch, pike, pollock, salmon, sardine, shark, snapper, sole, stingray, swordfish, tilapia, trout, tuna, and walleye, or combinations thereof.

9. The method of claim 1, further comprising aligning said collagen-producing cells to form a pattern.

10. The method of claim 1, wherein forming comprises automated deposition of multicellular bodies to form the sheets.

11. The method of claim 1, wherein each said layer of the plurality of sheets is **characterized by** a thickness adapted to allow diffusion to sufficiently support the maintenance and growth of said collagen-producing cells in culture.

12. The method of claim 1, wherein the thickness of each said sheet is about 50 µm to about 200 µm.

13. The method of claim 1, wherein the thickness of each said layer is:
50 µm to 150 µm; or
50 µm to 100 µm.

14. The method of claim 1, wherein said plurality of layers comprises:
2 to 50 layers; or
2 to 40 layers; or
2 to 30 layers.

15. The method of claim 1, further comprising dying the body using one or more colorants or pigments.

## Patentansprüche

1. Verfahren, um gezüchtetes Leder zu produzieren, wobei das Verfahren Folgendes umfasst:
Kultivieren einer oder mehrerer Arten von Kollagen-produzierenden Zellen *in vitro*;
Bilden einer Vielzahl von Blättern aus extrazellulärer Matrix, die von der einen oder mehreren Arten von Kollagen-produzierenden Zellen produziertes Kollagen enthalten;
Schichten der Vielzahl von Blättern, um einen Körper mit einem Volumen zu bilden; und
Verarbeiten des Körpers durch Gerben;
wobei ein Bilden der Vielzahl von Blättern ein Bilden einer Vielzahl von ebenen Schichten umfasst, umfassend ein angrenzendes Anordnen einer Vielzahl von länglichen mehrzelligen Körpern, wobei die besagten länglichen mehrzelligen Körper verschmolzen sind, um eine ebene Schicht zu bilden.

2. Verfahren nach Anspruch 1, wobei ein Anordnen ein Platzieren von mehrzelligen Körpern auf ein Trägersubstrat umfasst, das den mehrzelligen Körpern ermöglicht, zu verschmelzen, um eine im Wesentlichen ebene Schicht zu bilden;
optional wobei das Trägersubstrat für Flüssigkeiten und Nährstoffe durchlässig ist, um zu ermöglichen, dass Zellkulturmedien in Kontakt mit allen Oberflächen der besagten Schicht zu kommen.

3. Verfahren nach Anspruch 1, wobei:
ein Bilden eine automatisierte Ablagerung von mehrzelligen Körpern in die besagten Schichten ohne Strukturgerüst umfasst; oder
besagte mehrzellige Körper horizontal und/oder vertikal aneinander angrenzend angeordnet sind; oder
besagte Verschmelzung über 2 Stunden bis 24 Stunden erfolgt.

4. Verfahren nach Anspruch 1, wobei besagte längliche mehrzellige Körper:
aus Kollagen-produzierenden Zellen bestehen und die gleiche Länge aufweisen; oder
eine Länge im Bereich von 1 mm bis 10 m aufweisen; oder
eine Länge im Bereich von 1 cm bis 1 m aufweisen; oder
eine Länge im Bereich von 1 cm bis 50 cm aufweisen.

5. Verfahren nach Anspruch 1, ferner umfassend ein Verarbeiten des Körpers unter Verwendung eines oder mehrerer zusätzlichen Verarbeitungsschritte, optional wobei der zusätzliche Verarbeitungsschritt aus der Gruppe bestehend aus Konservieren, Einweichen, Beizen, Einlegen, Abbeizen, Ausdünnen, Nachgerben, Schmieren, Verhärten, Befeuchten, Abwelken, Schaben, Nachverchromen, Neutralisieren, Färben, Fetten, Füllen, Abziehen, Stopfen, Bleichen, Fixieren, Aushärten, Trocknen, Vorbehandeln, Fräsen, Stollen, Polieren, Zurichten, Einölen, Bürsten, Polstern, Imprägnieren, Sprühen, Walzbeschichten, Vorhanggießen, Abschleifen, Beschichten, Prägen, Bügeln, Glanzstoßen und Walken ausgewählt ist.

6. Verfahren nach Anspruch 1, wobei:
besagte Kollagen-produzierende Zellen eines oder mehrere von Folgenden umfassen: Epithelzellen, Fibroblasten, Keratinozyten, Korneozyten, Melanozyten, Langerhans-Zellen, Basalzellen oder eine Kombination davon; oder
besagte Epithelzellen Plattenepithel, Kuboidzellen, hochprismatische Zellen, Basalzellen oder eine Kombination davon umfassen; oder
besagte Fibroblasten dermale Fibroblasten sind; oder
besagte Keratinozyten epitheliale Keratinozyten, basale Keratinozyten, wuchernde basale Keratinozyten, differenzierte suprabasale Keratinozyten oder eine Kombination davon sind; oder
besagte Kollagen-produzierenden Zellen im Wesentlichen freie oder nicht aufgeteilte Keratinozyten, Fibroblasten oder Epithelzellen sind.

7. Verfahren nach Anspruch 1, wobei ein Bilden der Vielzahl von Blättern ein Bilden einer Vielzahl von Blättern der einen oder mehreren Arten von Kollagen-produzierenden Zellen und extrazellulärem Matrixmaterial umfasst, einschließlich Kollagen und ein oder mehrere Elemente ausgewählt aus der Gruppe bestehend aus: Keratin, Elastin, Gelatine, Proteoglykan, Dermatansulfatproteoglykan, Fibronektin, Laminin, Dermatopontin, Lipid, Fettsäure, Kohlenhydrat und eine Kombination davon.

8. Verfahren nach Anspruch 1, wobei besagte Kollagen-produzierende Zellen von Folgenden abgeleitet sind:
Säugetieren, die aus der Gruppe bestehend aus Antilope, Bär, Biber, Bison, Wildschwein, Kamel, Rentier, Katze, Rind, Hirsch, Hund, Elefant, Wapiti, Fuchs, Giraffe, Ziege, Hase, Pferd, Steinbock, Känguru, Löwe, Lama, Luchs, Nerz, Elch, Ochse, Pekari, Schwein, Kaninchen, Robbe, Schaf, Eichhörnchen, Tiger, Wal, Wolf, Yak und Zebra und einer Kombination davon ausgewählt sind; oder
Reptilien, die aus der Gruppe bestehend aus Schildkröte, Schlange, Krokodil und Alligator oder Kombinationen davon ausgewählt sind; oder
Vögel, die aus der Gruppe bestehend aus Huhn, Ente, Emu, Gans, Raufußhuhn, Strauß, Fasan, Taube, Wachtel und Truthahn oder Kombinationen davon ausgewählt sind; oder
Fische, die aus der Gruppe bestehend aus Sardelle, Barsch, Welsartigen, Karpfen, Kabeljau, Aal, Flunder, Fugu, Zackenbarsch, Schellfisch, Heilbutt, Hering, Makrele, Gemeine Goldmakrele, Mantarochen, Speerfisch, Granatbarsch, Flussbarsch, Hecht, Köhler, Lachs, Sardine, Hai, Schnapper, Seezunge, Stechrochen, Schwertfisch, Buntbarsch, Forelle, Thunfisch und Glasaugenbarsch oder Kombinationen davon ausgewählt sind.

9. Verfahren nach Anspruch 1, ferner umfassend ein Ausrichten besagter Kollagenproduzierender Zellen, um ein Muster zu bilden.

10. Verfahren nach Anspruch 1, wobei ein Bilden eine automatisierte Ablagerung von mehrzelligen Körpern umfasst, um die Blätter zu bilden.

11. Verfahren nach Anspruch 1, wobei jede besagte Schicht der Vielzahl von Blättern durch eine Stärke gekennzeichnet ist, die geeignet ist, um eine Diffusion zu ermöglichen, um den Fortbestand und das Wachstum der besagten Kollagen-produzierenden Zellen in der Kultur ausreichend zu unterstützen.

12. Verfahren nach Anspruch 1, wobei die Stärke von jedem besagten Blatt etwa 50 µm bis etwa 200 µm beträgt.

13. Verfahren nach Anspruch 1, wobei die Stärke von jeder besagten Schicht wie folgt ist:
50 µm bis 150 µm; oder
50 µm bis 100 µm.

14. Verfahren nach Anspruch 1, wobei die Vielzahl von Schichten Folgendes umfasst:
2 bis 50 Lagen; oder
2 bis 40 Lagen; oder
2 bis 30 Lagen.

15. Verfahren nach Anspruch 1, ferner umfassend ein Färben des Körpers unter Verwendung von einem oder mehreren Farbstoffen oder Pigmenten.

## Revendications

1. Procédé de production d'un cuir de synthèse, le procédé comprenant :
la culture d'au moins un type de cellules produisant du collagène *in vitro* ;
la formation d'une pluralité de feuilles de matrice extracellulaire comprenant un collagène produit par l'au moins un type de cellules produisant du collagène ;
la superposition de la pluralité de feuilles pour former un corps présentant un volume ; et
le traitement du corps par le tannage ;
la formation de la pluralité de feuilles comprenant la formation d'une pluralité de couches planes comprenant l'agencement adjacent d'une pluralité de corps multicellulaires allongés, lesdits corps multicellulaires allongés étant fusionnés pour former une couche plane.

2. Procédé selon la revendication 1, dans lequel l'agencement comprend le placement de corps multicellulaires sur un substrat de support qui permet aux corps multicellulaires de fusionner pour former une couche sensiblement plane.
le substrat de support étant éventuellement perméable aux fluides et nutriments pour permettre au milieu de culture cellulaire d'entrer en contact avec toutes les surfaces de ladite couche.

3. Procédé selon la revendication 1, dans lequel :
la formation comprend un dépôt automatique de corps multicellulaires dans lesdites couches sans échafaudage structurel ; ou
lesdits corps multicellulaires sont agencés horizontalement et/ou verticalement de manière adjacente les uns aux autres ; ou
ladite fusion a lieu pendant 2 heures à 24 heures.

4. Procédé selon la revendication 1, dans lequel lesdits corps multicellulaires allongés :
sont des cellules produisant du collagène et présentent les mêmes longueurs ; ou
présentent une longueur allant de 1 mm à 10 m ; ou
présentent une longueur allant de 1 cm à 1 m ; ou
présentent une longueur allant de 1 cm à 50 cm.

5. Procédé selon la revendication 1, comprenant en outre le traitement du corps à l'aide d'au moins une étape de traitement complémentaire, l'étape de traitement complémentaire étant éventuellement choisie dans le groupe constitué par la conservation, la trempe, le confitage, le picklage, le dépicklage, l'amincissement, le retannage, la lubrification, l'encroûtement, le trempage, l'essorage, le dérayage, le rechromage, la neutralisation, la teinture, la nourriture en bain, le remplissage, le dégommage, le rembourrage, le blanchiment, la fixation, le séchage initial, le séchage, le conditionnement, le foulage, le palissonnage, le lissage, le finissage, le graissage, le brossage, le foulardage, l'imprégnation, la pulvérisation, l'enduction au rouleau, l'enduction en rideau, le polissage, le placage, l'estampage, le repassage, le vernissage et le tonnelage.

6. Procédé selon la revendication 1, dans lequel :
lesdites cellules produisant du collagène comprennent des cellules épithéliales, des fibroblastes, des kératinocytes, des cornéocytes, des mélanocytes, des cellules de Langerhans, des cellules basales ou une combinaison de ceux-ci ; ou
lesdites cellules épithéliales comprennent des cellules squameuses, des cellules cuboïdes, des cellules colonnaires, des cellules basales ou une combinaison de celles-ci ; ou
lesdits fibroblastes sont des fibroblastes dermiques ; ou
lesdits kératinocytes sont des kératinocytes épithéliaux, des kératinocytes basaux, la prolifération de kératinocytes basaux, de kératinocytes supra-basaux différenciés ou une combinaison de ceux-ci ; ou
lesdites cellules produisant du collagène sont sensiblement libres de kératinocytes non différenciés, de fibroblastes ou de cellules épithéliales.

7. Procédé selon la revendication 1, dans lequel la formation de la pluralité de feuilles comprend la formation d'une pluralité de feuilles d'au moins un type de cellule produisant du collagène et de matériau de matrice extracellulaire comprenant du collagène et au moins un composant choisi dans le groupe constitué par : kératine, élastine, gélatine, protéoglycane, protéoglycane de sulfate de dermatane, glycosaminoglycane, fibronectine, laminine, dermatopontine, lipide, acide gras, glucide et une combinaison de ceux-ci.

8. Procédé selon la revendication 1, dans lequel lesdites cellules produisant du collagène proviennent de :
mammifères choisis dans le groupe constitué par l'antilope, l'ours, le castor, le bison, le sanglier, le chameau, le caribou, le chat, les bovins, le chevreuil, le chien, l'éléphant, le wapiti, le renard, la girafe, la chèvre, le lièvre, le cheval, le bouquetin, le kangourou, le lion, le lama, le lynx, le vison, l'élan, les boeufs, le pécari, le cochon, le lapin, le phoque, le mouton, l'écureuil, le tigre, la baleine, le loup, le yack et le zèbre et une combinaison de ceux-ci ; ou
des reptiles choisis dans le groupe constitué par la tortue, le serpent, le crocodile et l'alligator ou des combinaisons de ceux-ci ; ou
des oiseaux choisis dans le groupe constitué par la poule, le canard, l'émeu, l'oie, la grouse, l'autruche, le faisant, le pigeon, la caille et la dinde ou des combinaisons de ceux-ci ; ou
le poisson choisi dans le groupe constitué par l'anchoi, le bar, le poisson-chat, la carpe, la morue, l'anguille, la limande, le fugu, le mérou, l'aiglefin, le flétan, le hareng, le maquereau, le mahi mahi, la raie manta, le makaire, l'hoplostète orange, la perche, le brochet, la goberge, le saumon, la sardine, le requin, le vivaneau, la sole, la raie, l'espadon, le tilapia, la truite, le thon et le doré jaune ou des combinaisons de ceux-ci.

9. Procédé selon la revendication 1, comprenant en outre l'alignement desdites cellules produisant du collagène pour former un motif.

10. Procédé selon la revendication 1, dans lequel la formation comprend le dépôt automatique de corps multicellulaires pour former les feuilles.

11. Procédé selon la revendication 1, dans lequel chacune desdites couches de la pluralité de feuilles est **caractérisée par** une épaisseur conçue pour permettre la diffusion pour supporter suffisamment le maintien et la croissance desdites cellules produisant du collagène dans la culture.

12. Procédé selon la revendication 1, dans lequel l'épaisseur de chacune desdites feuilles va d'environ 50 µm à environ 200 µm.

13. Procédé selon la revendication 1, dans lequel l'épaisseur de chacune desdites couches va :
de 50 µm à 150 µm ; ou
de 50 µm à 100 µm.

14. Procédé de la revendication 1, dans lequel ladite pluralité de couches comprend :
de 2 à 50 couches ; ou
de 2 à 40 couches ; ou
de 2 à 30 couches.

15. Procédé de la revendication 1, comprenant en outre la teinture du corps à l'aide d'au moins un colorant ou pigment.
